# FASCICULE DE BREVET EUROPEEN

(11) **EP 1 987 808 B1**
(45) Date de publication et mention de la délivrance du brevet: **21.07.2010**
(21) Numéro de dépôt: 08155207.7
(22) Date de dépôt: 25.04.2008
(51) Int. Cl.: A61K 8/02, A61Q 1/14, A61Q 19/10, A61K 8/06, A61K 8/04

(54) **Composition de soin et/ou de démaquillage de matière(s) kératinique(s)**
Pflege- und/oder Abschminkzusammensetzung zum Abschminken von keratinhaltigen Stoffen
Composition for care and/or make-up removal of keratinous material(s)

(30) Priorité: 27.04.2007 FR 0754749
(43) Date de publication de la demande: 05.11.2008
(73) Titulaire: L'Oréal, 75008 Paris (FR)
(72) Inventeur: Compain, Delphine, 75013, Paris (FR)
(74) Mandataire: Le Coupanec, Pascale A.M.P.

(56) Documents cités:
- EP-A- 1 516 613
- EP-A- 1 704 851
- US-A- 5 612 307

## Description

La présente invention vise à proposer des compositions destinées plus particulièrement au soin et/ou au démaquillage des matières kératiniques.

Le nettoyage des matières kératiniques, et plus particulièrement de la peau, est très important. Ainsi, au niveau du visage par exemple, il se doit d'être le plus efficace possible car les résidus gras, tels que l'excès de sébum, et les résidus de produits cosmétiques utilisés quotidiennement, tels que les produits de maquillage, ont tendance à s'accumuler dans les replis cutanés et à la surface de la peau. Ils peuvent ainsi obstruer des pores de la peau et entraîner l'apparition de boutons.

On peut distinguer les produits de nettoyage de la peau conventionnels en trois familles principales :
- les lotions et les gels aqueux détergents moussants,
- les crèmes et laits démaquillants, et
- les huiles et les gels anhydres nettoyants rinçables.

Les lotions et les gels aqueux détergents moussants ont une action nettoyante efficace grâce aux tensioactifs qu'ils contiennent. Ils sont en outre agréables cosmétiquement du fait qu'ils moussent et qu'ils sont facilement éliminés. Toutefois, dans la mesure où ils ne contiennent pas d'huiles cosmétiques, ils peuvent présenter l'inconvénient d'assécher la peau par leur action délipidante.

Les crèmes et les laits démaquillants contiennent quant à eux à la fois des huiles, des émulsionnants et des tensioactifs détergents, ces derniers étant en quantité suffisamment faible pour ne pas déstabiliser l'émulsion. Malheureusement, ces produits, en dépit de leur bonne efficacité, ne sont pas moussants et présentent donc une rinçabilité insuffisante. Ils requièrent généralement l'emploi conjoint d'une lotion détergente pour parfaire le rinçage.

Les compositions huileuses sont également reconnues pour leur efficacité comme nettoyant et/ou démaquillant. Elles permettent en effet de dissoudre très facilement les salissures lipophiles et le maquillage, notamment le maquillage sans transfert, réputé difficile à démaquiller. Ces produits sont efficaces et généralement bien tolérés. Ils présentent toutefois l'inconvénient de ne pas mousser et de ne pas conférer de sensation de fraîcheur à l'application. Quant aux compositions huileuses ayant l'aspect d'un gel, elles sont généralement épaissies par des cires, des silices ou encore des argiles qui leur confèrent un aspect habituellement trouble ou opaque et donc pas attrayant.

En conséquence, il demeure à ce jour un besoin d'un produit de nettoyage et/ou de démaquillage qui réunit les avantages des huiles ou huiles émulsionnées, notamment en terme d'innocuité et d'aspect non desséchant, et ceux des gels moussants, notamment en terme de performance de nettoyage, sans reproduire par ailleurs leurs inconvénients respectifs.

La présente invention a précisément pour objet de proposer un nouveau type de produit de nettoyage et/ou démaquillage permettant de donner satisfaction en ces termes.

Ainsi, la présente invention concerne, à titre principal, un produit cosmétique de nettoyage et/ou de démaquillage de matière(s) kératinique(s) comprenant, de manière séparée l'une de l'autre, au moins une première et une deuxième compositions cosmétiques et dans lequel :
- la première composition A se présente sous la forme d'une émulsion huile-dans-eau obtenue selon le procédé d'inversion de phase en température selon technologie PIT « Phase Inversion Température » et comprend, dans un milieu physiologiquement acceptable, au moins 50 % en poids d'au moins une huile ou une phase huileuse, par rapport au poids total de ladite première composition, et
- la deuxième composition B se présente sous la forme d'un gel aqueux et comprend, dans un milieu physiologiquement acceptable, au moins un tensioactif moussant.

Le produit selon l'invention peut notamment constituer un produit nettoyant, démaquillant, un produit de gommage, un produit exfoliant.

Selon une variante de réalisation, la deuxième composition B se présente sous la forme d'un gel aqueux transparent.

Selon une autre variante de réalisation, les première et deuxième compositions présentent des viscosités approchantes.

Selon un mode de réalisation particulier, les première et deuxième compositions sont conditionnées séparément l'une dans l'autre, mais réunies dans un unique ensemble de conditionnement.

Selon un autre mode de réalisation, les première et deuxième compositions sont conditionnées séparément l'une de l'autre dans des ensembles de conditionnement distincts.

La présente invention a en outre pour objet, un ensemble de conditionnement et de distribution des compositions formant un produit selon l'invention, ledit ensemble comprenant au moins deux compartiments indépendants comprenant respectivement chacune desdites compositions et étant ajusté à la distribution des deux compositions de manière séparée ou en mélange.

Selon une variante de réalisation, le mélange des compositions est effectué au sein dudit ensemble juste avant usage et donc avant application sur au moins une matière kératinique.

Selon une autre variante de réalisation, le mélange des deux compositions est réalisé de manière extemporanée à l'extérieur dudit ensemble soit sur un support dédié au nettoyage et/ou au démaquillage, tel un coton par exemple, soit directement sur la matière kératinique devant faire l'objet du nettoyage et/ou démaquillage.

La présente invention vise également, selon un autre de ses aspects, un procédé de nettoyage et/ou démaquillage de matière(s) kératinique(s) comprenant au moins les étapes consistant à :
a) disposer d'une première composition A se présentant sous la forme d'une émulsion huile-dans-eau obtenue selon le procédé d'inversion de phase en température selon la technologie PIT et comprenant, dans un milieu physiologiquement acceptable, au moins 50 % en poids d'au moins une huile ou phase huileuse par rapport au poids total de ladite première composition,
b) disposer d'une seconde composition B se présentant sous la forme d'un gel aqueux et comprenant, dans un milieu physiologiquement acceptable, au moins un tensioactif moussant,
c) procéder à la mise en contact extemporanée de ladite première composition A avec ladite seconde composition B, et
d) appliquer le mélange obtenu à l'étape précédente sur ladite matière kératinique.

Selon une variante de réalisation, les étapes c) et d) sont réalisées simultanément.

Selon une autre variante de réalisation, l'étape d) est réalisée en présence d'eau.

Le cas échéant, l'étape d) peut être suivie avantageusement d'une étape de rinçage avec de l'eau de la matière kératinique ainsi traitée.

Plus particulièrement, ladite matière kératinique est la peau ou la chevelure.

Plus préférentiellement, il s'agit de la peau.

Le produit conforme à la présente invention s'avère particulièrement avantageux dans la mesure où il permet de cumuler les avantages respectifs des compositions de nettoyage et/ou maquillage conventionnelles tout en s'affranchissant de leurs défauts respectifs.

Ses performances de démaquillage et/ou nettoyage s'avèrent significativement accrues comparativement à une unique composition conventionnelle.

En particulier, ce produit, qui ne requiert la mise en contact des deux compositions le formant qu'au moment de leur utilisation, c'est-à-dire de manière extemporanée, s'avère doté d'un pouvoir moussant amélioré par rapport à celui observé avec une unique composition formée à partir des composants respectifs de la première et seconde compositions selon l'invention. Notamment la mousse est plus onctueuse, plus blanche et à fines bulles.

Comme précisé précédemment, au moins l'une des compositions formant le produit selon l'invention, dite encore première composition, comprend au moins une huile ou une phase huileuse.

### PREMIERE COMPOSITION A

La première composition A comprend au moins 50% en poids d'au moins une huile ou une phase huileuse, par rapport à son poids total.

Elle peut notamment comprendre au moins 55% en poids, par exemple au moins 60% en poids, voire au moins 65% en poids d'au moins une huile ou une phase huileuse, par rapport à son poids total.

Au sens de l'invention, la première composition A peut comprendre une unique huile ou un mélange de plusieurs huiles désigné par le terme de phase huileuse.

On entend par "huile" un corps gras liquide à la température ambiante (25 °C).

Comme huiles utilisables dans la composition de l'invention, on peut citer par exemple :
- les huiles hydrocarbonées d'origine animale (poisson), telles que le perhydrosqualène et le squalane ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment les esters et éthers d'acides gras, comme les huiles de formules R¹ COOR² et R¹ OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle, l'isononanoate d'isnonyle; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle (Prisorine 3631) ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de Parléam;
- les huiles de silicone, comme par exemple les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane et la cyclopentasiloxane ; les polydiméthylsiloxanes (ou diméthicones) comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsilo xanes;
- les alcools gras ayant de 8 à 26 atomes de carbone, comme l'alcool cétylique, l'alcool stéarylique et leur mélange (alcool cétylstéarylique);
- les huiles fluorées partiellement hydrocarbonées et/ou siliconées comme celles décrites dans le document JP-A-2-295912 ;
- et leurs mélanges.

On entend ci-dessus par « huile hydrocarbonée », toute huile comportant majoritairement des atomes de carbone et d'hydrogène, et éventuellement des groupements ester, éther, fluoré, acide carboxylique et/ou alcool.

Selon un mode préféré de réalisation de l'invention, la composition A contient au moins une huile choisie parmi le polyisobutène hydrogéné (nom INCI : Hydrogenated Polyisobutene ou C13-16 Isoparaffin) comme le produit commercialisé sous la dénomination Parleam par la société NOF Corporation, et les esters d'acide gras comme notamment l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle, le stéarate d'éthyl-2-hexyle.

Les huiles préférées sont les esters d'alcools gras tels que le palmitate d'éthyl-2-hexyle et le stéarate d'éthyl-2-hexyle.

Cette huile, ou encore phase huileuse, formée à partir d'une ou plusieurs des huiles précitées, le cas échéant en mélange avec d'autres corps gras et constituants lipophiles, constitue la phase huileuse d'une émulsion huile-dans-eau.

Selon un mode de réalisation de l'invention, la première composition A comprend au moins un émulsionnant.

Ces émulsions contiennent ainsi généralement au moins un tensioactif notamment choisi parmi les émulsionnants amphotères, anioniques, cationiques ou non ioniques, utilisés seuls ou en mélange. Les émulsionnants sont choisis de manière appropriée suivant la phase continue de l'émulsion à obtenir (E/H ou H/E). Quand l'émulsion est multiple, elle comporte généralement un émulsionnant dans l'émulsion primaire et un émulsionnant dans la phase externe dans laquelle est introduite l'émulsion primaire.

Comme émulsionnants utilisables pour la préparation des émulsions E/H, on peut citer par exemple les alkyl esters ou éthers de sorbitan, de glycérol ou de sucres ; les tensioactifs siliconés comme les dimethicone copolyols tels que le mélange de cyclomethicone et de dimethicone copolyol, vendu sous les dénominations DC 5225 C et DC 3225 C par la société Dow Corning, et comme les alkyl-dimethicone copolyols tels que le Laurylmethicone copolyol vendu sous la dénomination « Dow Corning 5200 Formulation Aid » par la société Dow Corning, le Cetyl dimethicone copolyol vendu sous la dénomination Abil EM 90^{R} par la société Goldschmidt et le mélange de Polyglyceryl-4 isostearate/Cetyl dimethicone copolyol/Hexyl laurate vendu sous la dénomination Abil WE 09^{R} par la société Goldschmidt. On peut y ajouter aussi un ou plusieurs co-émulsionnants, qui, de manière avantageuse, peuvent être choisis dans le groupe comprenant les esters d'acide gras à chaîne ramifiée et de polyol, et notamment les esters d'acide gras à chaîne ramifiée et de glycérol et/ou de sorbitan et par exemple l'isostéarate de polyglycéryle, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Comme émulsionnants utilisables pour la préparation des émulsions H/E, on peut citer par exemple les émulsionnants non ioniques tels que les esters d'acides gras et de polyols oxyalkylénés (plus particulièrement polyoxyéthylénés), et par exemple les stéarates de polyéthylène glycol comme le stéarate de PEG-100, le stéarate de PEG-50 et le stéarate de PEG-40 ; et leurs mélanges tels que le mélange de mono-stéarate de glycéryle et de stéarate de polyéthylène glycol (100 OE) commercialisé sous la dénomination SIMULSOL 165 par la société SEPPIC ; les esters d'acides gras et de sorbitan oxyalkylénés comprenant par exemple de 20 à 100 OE, et par exemple ceux commercialisés sous les dénominations commerciales Tween 20 ou Tween 60 par la société Uniqema ; les éthers d'alcools gras oxyalkylénés (oxyéthylénés et/ou oxypropylénés) ; les esters de sucres comme le stéarate de sucrose ; et leurs mélanges comme par exemple le mélange de stéarate de glycéryle et de stéarate de PEG-100, commercialisé sous la dénomination Arlacel 165 par la société Uniqema.

On peut ajouter à ces émulsionnants, des co-émulsionnants tels que par exemple les alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique.

On peut aussi préparer des émulsions sans tensioactifs émulsionnants ou en contenant moins de 0,5 % du poids total de la composition, en utilisant des composés appropriés, par exemple les polymères ayant des propriétés émulsionnantes tels que les polymères commercialisés sous les dénominations Carbopol 1342 et Pemulen par la société Noveon ; ou les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société CLARIANT sous la dénomination « Hostacerin AMPS » (nom INCI : ammonium polyacryldimethyltauramide) ou comme le polymère en émulsion commercialisé sous la dénomination Sepigel 305 par la société Seppic (nom INCI : Polyacrylamide / C13-C14 isoparaffine / laureth-7) ; les particules de polymères ioniques ou non ioniques, plus particulièrement des particules de polymère anionique comme notamment les polymères d'acide isophtalique ou d'acide sulfoisophtalique, et en particulier les copolymères de phtalate / sulfoisophtalate / glycol (par exemple diéthylèneglycol / Phtalate / isophtalate/1,4-cyclohexane-diméthanol (nom INCI : Diglycol/ CHDM/I sophtalates/ SIP Copolymer) vendus sous les dénominations Eastman AQ polymer (AQ35S, AQ38S, AQ55S, AQ48 Ultra) par la société Eastman Chemical.

L'émulsion est préparée par la technique d'inversion de phase en température (émulsions PIT). Cette technique permet notamment d'accéder à une taille moyenne des globules constituant la phase huileuse variant de 0,1 à 4 µm (100 à 4000 nm).

Le principe d'émulsification par inversion de phase en température (en Anglais : Phase Inversion Temperature ou PIT) est bien connu de l'homme de l'art ; il a été décrit en 1968 par K. Shinoda (J. Chem. Soc. Jpn., 1968, 89, 435). Il a été montré que cette technique d'émulsification permet d'obtenir des émulsions fines stables (K. Shinoda et H. Saito, J. Colloïd Interface Sci., 1969,30, 258). Cette technologie a été appliquée en cosmétique dès 1972 par Mitsui *et al.* («Application of the phase-inversion-temperature method to the emulsification of cosmetics» ; T. Mitsui, Y. Machida and F. Harusawa, American. Cosmet. Perfum., 1972, 87, 33).

L'émulsification par inversion de phase est expliquée en détail dans l'ouvrage T. Förster, W. von Rybinski, A. Wadle, Influence of microemulsion phases on the préparation of fine disperse emulsions, Advances in Colloid and interface sciences, 58, 119-149, 1995 cité ici pour référence.

Le principe de cette technique est le suivant : on prépare une émulsion E/H (introduction de la phase aqueuse dans la phase huileuse) à une température qui doit être supérieure à la température d'inversion de phase du système, c'est à dire la température à laquelle l'équilibre entre les propriétés hydrophile et lipophile du ou des émulsionnants mis en oeuvre est atteint ; à température élevée, c'est-à-dire supérieure à la température d'inversion de phase (>PIT), l'émulsion est de type eau-dans-huile, et, au cours de son refroidissement, cette émulsion s'inverse à la température d'inversion de phase, pour devenir une émulsion de type huile-dans-eau, et ceci en étant passée auparavant par un état de microémulsion. Ce procédé permet d'obtenir facilement des émulsions de diamètre généralement inférieur à 4 µm.

Plus précisément, les émulsions PIT peuvent être obtenues par un procédé d'inversion de phase réalisé comme suit :
- Peser dans un récipient tous les constituants de la composition (à l'exception des matières premières thermosensibles s'il y en a).
- Homogénéiser le mélange, par exemple au moyen d'un Rayneri 350 tours/min, et chauffer en augmentant progressivement la température au moyen d'un bain marie jusqu'à une température supérieure ou égale à la température d'inversion de phase T2, c'est-à-dire jusqu'à l'obtention d'une phase transparente ou translucide (zone de microémulsion ou de phase lamellaire) puis d'une phase blanche plus visqueuse qui indique l'obtention de l'émulsion inverse (E/H).
- Arrêter le chauffage et maintenir l'agitation jusqu'à retour à la température ambiante, en passant par la température d'inversion de phase T1, c'est-à-dire la température à laquelle se forme une émulsion H/E fine.
- Lorsque la température est redescendue en dessous de la zone d'inversion de Phase en Température (T1), additionner éventuellement les matières premières thermosensibles.

On obtient une émulsion H/E stable dont les gouttelettes d'huile sont fines.

Dans le procédé précité, l'ajout des matières premières non thermosensibles, telles que les charges telles que la silice, les colorants, les polymères, les parfums, est généralement réalisé en fin de protocole.

Dans la zone de formation d'une microémulsion (mélange translucide), les interactions hydrophiles et hydrophobes sont équilibrées car la tendance du tensioactif est de former aussi bien des micelles directes que des micelles inverses. Par chauffage au-delà de cette zone, il y a formation d'une émulsion E/H (mélange opaque blanc), car le tensioactif favorise la formation d'une émulsion eau dans huile. Puis lors du refroidissement en dessous de la zone d'inversion de phase, l'émulsion devient une émulsion H/E.

Les tensioactifs émulsionnants du type huile-dans-eau habituellement utilisés ont un HLB (HLB = Hydrophilic Lipophilic Balance) allant de 8 à 18. Ces émulsionnants, grâce à leur structure amphiphile, se placent à l'interface phase huileuse / phase aqueuse, et stabilisent ainsi les gouttelettes d'huiles dispersées.

Le système émulsionnant utilisé dans la première composition A selon l'invention peut comprendre un ou plusieurs émulsionnants dont la solubilité dans l'huile augmente avec l'augmentation de la température, émulsionnants permettant d'obtenir des émulsions par inversion de phase en température. Le HLB (hydrophilic lipophilic balance) de ces émulsionnants va de 8 à 18 et de préférence de 10 à 16, et ces émulsionnants sont choisis parmi les alcools gras éthoxylés, les acides gras éthoxylés, les glycérides partiels d'acides gras éthoxylés, les triglycérides d'acides gras polyglycérolés et leurs dérivés éthoxylés, et leurs mélanges.

Les émulsionnants sont de préférence choisis parmi les alcools gras éthoxylés ou les acides gras éthoxylés ayant les formules (I) et (II) suivantes :

R-O-(CH₂-CH₂-O)ₘH (I)

R-COO-(CH₂-CH₂-O)ₘH (II)

où R est une chaîne hydrocarbonée linéaire ou ramifiée, saturée ou insaturée, ayant de 10 à 24 atomes de carbone, et m est un nombre entier allant de 8 à 50.

Comme alcools gras éthoxylés, on peut citer par exemple les produits d'addition d'oxyde d'éthylène avec l'alcool laurylique, notamment ceux comportant de 9 à 50 groupes oxyéthylénés (Laureth-9 à Laureth-50 en noms INCI) ; les produits d'addition d'oxyde d'éthylène avec l'alcool béhénylique, notamment ceux comportant de 9 à 50 groupes oxyéthylénés (Beheneth-9 à Beheneth-50 en noms INCI) ; les produits d'addition d'oxyde d'éthylène avec l'alcool cétéarylique (mélange d'alcool cétylique et d'alcool stéarylique), notamment ceux comportant de 9 à 30 groupes oxyéthylénés (Ceteareth-9 à Ceteareth-30 en noms INCI) ; les produits d'addition d'oxyde d'éthylène avec l'alcool cétylique, notamment ceux comportant de 9 à 30 groupes oxyéthylénés (Ceteth-9 à Ceteth-30 en noms INCI) ; les produits d'addition d'oxyde d'éthylène avec l'alcool stéarylique, notamment ceux comportant de 9 à 30 groupes oxyéthylénés (Steareth-9 à Steareth-30 en noms INCI) ; les produits d'addition d'oxyde d'éthylène avec l'alcool isostéarylique, notamment ceux comportant de 9 à 50 groupes oxyéthylénés (Isosteareth-9 à Isosteareth-50 en noms INCI) ; et leurs mélanges.

Comme acides gras éthoxylés, on peut citer par exemple les produits d'addition d'oxyde d'éthylène avec les acides laurique, palmitique, stéarique ou béhénique, et leurs mélanges, notamment ceux comportant de 9 à 50 groupes oxyéthylénés tels que les laurates de PEG-9 à PEG-50 (en noms INCI : PEG-9 laurate à PEG-50 laurate) ; les palmitates de PEG-9 à PEG-50 (en noms INCI : PEG-9 palmitate à PEG-50 palmitate) ; les stéarates de PEG-9 à PEG-50 (en noms INCI : PEG-9 stearate à PEG-50 stearate) ; les palmito-stéarates de PEG-9 à PEG-50 ; les béhénates de PEG-9 à PEG-50 (en noms INCI : PEG-9 behenate à PEG-50 behenate) ; et leurs mélanges.

On peut utiliser aussi des mélanges de ces dérivés oxyéthylénés d'alcools gras et d'acides gras.

De préférence, le système émulsionnant d'une première composition de l'invention, se présentant sous la forme d'une émulsion obtenue selon la technique PIT, contient comme émulsionnant au moins un alcool gras éthoxylé, plus particulièrement les ceteth, ceteareth, beheneth, et leurs mélanges, et plus particulièrement le beheneth-10.

Ce système émulsionnant peut contenir en outre un ou plusieurs co-émulsionnants. Comme co-émulsionnants, on peut citer par exemple les alcools gras ayant 8 à 30 atomes de carbone, comme par exemple l'alcool cétylique, l'alcool stéarylique, l'alcool béhénique ; les acides gras ayant 8 à 30 atomes de carbone, comme par exemple l'acide palmitique, l'acide stéarique, l'acide béhénique ; les esters gras de glycérol, comme par exemple le glycéryl stéarate ; les dérivés oxyéthylénés de ces alcools gras, acides gras et esters gras de glycérol, comportant 2 à 8 groupes oxyde d'éthylène, et leurs mélanges.

Le système émulsionnant est présent en une quantité allant de 2 à 20 %, de préférence de 3 à 16 % et mieux de 3 à 11 % en poids par rapport au poids total de la première composition.

Le rapport système émulsionnant / phase lipophile peut aller par exemple de 0,04 à 0,2, et de préférence de 0,06 à 0,18.

### DEUXIEME COMPOSITION B

En ce qui concerne la seconde composition B, elle se caractérise, d'une part, par le fait qu'elle se présente sous la forme d'un gel aqueux et, d'autre part, en ce qu'elle contient au moins un tensioactif moussant.

Selon un mode de réalisation particulier, cette composition B se présente sous la forme d'un gel aqueux transparent.

Au sens de l'invention, le mot « transparent » signifie qu'au travers d'une bouteille transparente contenant ladite seconde composition, on peut distinguer les caractères imprimés sur une page du journal placée derrière cette bouteille.

La composition B contient au moins un tensioactif moussant. Toutefois, la composition A peut éventuellement contenir aussi un ou plusieurs tensioactifs moussants qui viennent compléter ceux présents dans la composition B.

En ce qui concerne la définition du tensioactif moussant, on peut se reporter au document « Encyclopédia of chemical technology, Kirkt-Othmer », volume 22, pages 333-432, 3ème édition 1979, Edition Wiley, où sont citées les principales classes de tensioactifs connues de l'homme du métier, ainsi que leur fonction, en particulier le fait d'être moussant.

Préférentiellement, ces tensioactifs peuvent être choisis parmi les tensioactifs non ioniques, anioniques, et amphotères.

### Tensioactifs moussants non ioniques

Les tensioactifs moussants non ioniques peuvent être choisis notamment parmi les alkyl polyglucosides (APG), les esters de glycérol oxyalkylénés, les esters de sucre oxyalkylénés, et leurs mélanges. Ce sont de manière préférée des APG.
1) Comme alkylpolyglucosides, on utilise de préférence ceux contenant un groupe alkyle comportant de 6 à 30 atomes de carbone et de préférence de 8 à 16 atomes de carbone, et contenant un groupe glucoside comprenant de préférence 1, 2 à 3 unités de glucoside. Les alkylpolyglucosides peuvent être choisis par exemple parmi le decylglucoside (Alkyl-C9/C11-polyglucoside (1.4)) comme le produit commercialisé sous la dénomination Mydol 10^{®} par la société Kao Chemicals ou le produit commercialisé sous la dénomination Plantacare 2000 UP^{®} par la société Cognis ; le caprylyl/capryl glucoside comme le produit commercialisé sous la dénomination Plantacare KE 3711^{®} par la société Cognis ; le laurylglucoside comme le produit commercialisé sous la dénomination Plantacare 1200 UP^{®} par la société Cognis ; le cocoglucoside comme le produit commercialisé sous la dénomination Plantacare 818 UP^{®} par la société Cognis ; le caprylylglucoside comme le produit commercialisé sous la dénomination Plantacare 810 UP^{®} par la société Cognis ; et leurs mélanges.
2) Les esters de glycérol oxyalkylénés sont notamment les dérivés polyoxyéthylénés des esters de glycéryle et d'acide gras et de leurs dérivés hydrogénés. Ces esters de glycérol oxyalkylénés peuvent être choisis par exemple parmi les esters de glycéryle et d'acides gras hydrogénés et oxyéthylénés tel que le PEG-200 hydrogenated glyceryl palmate commercialisé sous la dénomination Rewoderm LI-S 80 par la société Goldschmidt ; les cocoates de glycéryle oxyéthylénés comme le PEG-7 glyceryl cocoate commercialisé sous la dénomination Tegosoft GC par la société Goldschmidt, et le PEG-30 glyceryl cocoate commercialisé sous la dénomination Rewoderm LI-63 par la société Goldschmidt ; et leurs mélanges.
3) Les esters de sucres oxyalkylénés sont notamment les éthers de polyéthylène glycol des esters d'acide gras et de sucre. Ces esters de sucre oxyalkylénés peuvent être choisis par exemple parmi les esters de glucose oxyéthylénés tels que le PEG-120 methyl glucose dioleate commercialisé sous la dénomination Glucamate DOE 120 par la société Amerchol.

Selon un mode préféré de réalisation de l'invention, le tensioactif non ionique est un alkylpolyglucoside qui peut être choisi notamment parmi le decylglucoside, le caprylyl/capryl glucoside, le laurylglucoside, le cocoglucoside, le caprylylglucoside, et leurs mélanges.

### Tensioactifs anioniques

Les tensioactifs moussants anioniques peuvent être choisis notamment parmi les dérivés anioniques de protéines d'origine végétale ou de protéines de soie, les phosphates et alkylphosphates, les carboxylates, les sulfosuccinates, les dérivés des aminoacides, les alkyl sulfates, les alkyl éther sulfates, les sulfonates, les iséthionates, les taurates, les alkyl sulfoacétates, les polypeptides, les dérivés anioniques d'alkyl polyglucoside, et leurs mélanges.
1) Les dérivés anioniques de protéines d'origine végétale sont des hydrolysats de protéine à groupement hydrophobe, ledit groupement hydrophobe pouvant être naturellement présent dans la protéine ou être ajouté par réaction de la protéine et/ou de l'hydrolysat de protéine avec un composé hydrophobe. Les protéines sont d'origine végétale ou dérivées de soie, et le groupement hydrophobe peut être notamment une chaîne grasse, par exemple une chaîne alkyle comportant de 10 à 22 atomes de carbone. Comme dérivés anioniques de protéines d'origine végétale, on peut plus particulièrement citer les hydrolysats de protéines de pomme, de blé, de soja, d'avoine, comportant une chaîne alkyle ayant de 10 à 22 atomes de carbone et leurs sels. La chaîne alkyle peut être notamment une chaîne lauryle et le sel peut être un sel de sodium, de potassium et/ou d'ammonium.
   Ainsi, comme hydrolysats de protéine à groupement hydrophobe, on peut citer par exemple les sels des hydrolysats de protéine de soie modifiée par l'acide laurique, tels que le produit commercialisé sous la dénomination KAWA SILK par la société Kawaken ; les sels des hydrolysats de protéine de blé modifiée par l'acide laurique, tels que le sel de potassium commercialisé sous la dénomination Aminofoam W OR par la société Croda (nom INCI : Potassium lauroyl wheat aminoacids) et le sel de sodium commercialisé sous la dénomination PROTEOL LW 30 par la société Seppic (nom INCI : sodium lauroyl wheat aminoacids) ; les sels des hydrolysats de protéine d'avoine comportant une chaîne alkyle ayant de 10 à 22 atomes de carbone, et plus spécialement les sels des hydrolysats de protéine d'avoine modifiée par l'acide laurique, tels que le sel de sodium commercialisé sous la dénomination PROTEOL OAT (solution aqueuse à 30 %) par la société Seppic (nom INCI : Sodium lauroyl oat aminoacids) ; les sels des hydrolysats de protéine de pomme, comportant une chaîne alkyle ayant de 10 à 22 atomes de carbone, tels que le sel de sodium commercialisé sous la dénomination PROTEOL APL (solution hydroglycolique à 30%) par la société Seppic (nom INCI : Sodium Cocoyl Apple amino acids). On peut citer aussi le mélange de lauroyl-aminoacides (aspartique, glutamique, glycine, alanine) neutralisé au N-methylglycinate de sodium, commercialisé sous la dénomination PROTEOL SAV 50 S par la société Seppic (nom INCI : Sodium Cocoyl amino acids).
2) Comme phosphates et alkylphosphates, on peut citer par exemple les monoalkylphosphates et les dialkyl phosphates, tels que le mono-phosphate de lauryle commercialisé sous la dénomination MAP 20^{®} par la société Kao Chemicals, le sel de potassium de l'acide dodécyl-phosphorique, mélange de mono- et di-ester (diester majoritaire) commercialisé sous la dénomination CRAFOL AP-31^{®} par la société Cognis, le mélange de monoester et de di-ester d'acide octylphosphorique, commercialisé sous la dénomination CRAFOL AP-20^{®} par la société Cognis, le mélange de monoester et de diester d'acide phophorique de 2-butyloctanol éthoxylé (7 moles d'OE), commercialisé sous la dénomination ISOFOL 12 7 EO-PHOSPHATE ESTER^{®} par la société Condea, le sel de potassium ou de triéthanolamine de mono-alkyl (C₁₂-C₁₃) phosphate commercialisé sous les références ARLATONE MAP 230K-40^{®} et ARLATONE MAP 230T-60^{®} par la société Uniqema, le lauryl phosphate de potassium commercialisé sous la dénomination DERMALCARE MAP XC-99/09^{®} par la société Rhodia Chimie, et le cétylphosphate de potassium commercialisé sous la dénomination ARLATONE MAP 160K par la société Uniqema.
3) Comme carboxylates, on peut citer :
   - Les amido éthercarboxylates (AEC), comme le Lauryl amido ether carboxylate de sodium (3 OE), commercialisé sous la dénomination AKYPO FOAM 30^{®} par la société Kao Chemicals.
   - Les sels d'acides carboxyliques polyoxyéthylénés, comme le Lauryl ether carboxylate de sodium (C₁₂₋₁₄₋₁₆ 65/25/10) oxyéthyléné (6 OE) commercialisé sous la dénomination AKYPO SOFT 45 NV^{®} par la société Kao Chemicals, les acides gras d'origine huile d'olive polyoxyéthylénés et carboxyméthylés commercialisés sous la dénomination OLIVEM 400^{®} par la société BIOLOGIA E TECNOLOGIA, le tri-decyl ether carboxylate de sodium oxyéthyléné (6 OE) commercialisé sous la dénomination NIKKOL ECTD-6NEX^{®} par la société Nikkol.
   - Les sels d'acides gras ayant une chaîne alkyl en C₆ à C₂₂, neutralisés par une base organique ou minérale, qui constituent les savons. Le sel d'acide gras ou savon est obtenu à partir d'un acide gras et d'une base, l'acide gras comportant une chaîne alkyle linéaire ou ramifiée, saturée ou insaturée, ayant de 12 à 22 atomes de carbone et de préférence 12 à 20 atomes de carbone. Les bases (aussi appelées aussi agents de saponification) neutralisent totalement ou partiellement les acides gras. Les bases susceptibles d'être utilisées pour obtenir les sels peuvent être par exemple les bases minérales comme les hydroxydes de métaux alcalins (hydroxyde de sodium et potasse), les hydroxydes de métaux alcalino-terreux (de magnésium) ou l'hydroxyde d'ammonium, ou encore les bases organiques comme la triéthanolamine, la N-méthylglucamine, la lysine et l'arginine. L'acide gras peut être choisi en particulier parmi les acides gras en C₁₀ à C₂₄, et notamment en C₁₂-C₁₈, et en particulier l'acide laurique, l'acide myristique, l'acide stéarique, l'acide palmitique et leurs mélanges.
   Le savon est généralement introduit dans une seconde composition sous forme de la base d'une part et de l'acide gras d'autre part, la formation du sel se faisant in situ.
4) Comme dérivés des aminoacides, on peut citer notamment les sels alcalins d'aminoacides, tels que :
   - les sarcosinates, comme le Lauroyl sarcosinate de sodium commercialisé sous la dénomination SARKOSYL NL 97^{®} par la société Ciba ou commercialisé sous la dénomination ORAMIX L 30^{®} par la société Seppic, le myristoyl sarcosinate de sodium commercialisé sous la dénomination NIKKOL SARCOSINATE MN^{®} par la société Nikkol, le palmitoyl sarcosinate de sodium commercialisé sous la dénomination NIKKOL SARCOSINATE PN^{®} par la société Nikkol.
   - les alaninates, comme le N-lauroyl-N methyl amidopropionate de sodium commercialisé sous la dénomination SODIUM NIKKOL ALANINATE LN 30^{®} par la société Nikkol, ou commercialisé sous la dénomination ALANONE ALE^{®} par la société Kawaken, le N-lauroyl N-methyl alanine triéthanolamine commercialisé sous la dénomination ALANONE ALTA^{®} par la société Kawaken.
   - les glutamates, comme le mono-cocoyl glutamate de triéthanolamine commercialisé sous la dénomination ACYLGLUTAMATE CT-12^{®} par la société Ajinomoto, le lauroylglutamate de triéthanolamine commercialisé sous la dénomination ACYLGLUTAMATE LT-12^{®} par la société Ajinomoto.
   - les aspartates, comme le mélange de N-lauroyl aspartate de triethanolamine / N-myristoyl aspartate de triethanolamine commercialisé sous la dénomination ASPARACK^{®} par la société Mitsubishi.
   - les dérivés de glycine (glycinates), comme le N-cocoyl glycinate de sodium commercialisé sous les dénominations AMILITE GCS-12^{®} et AMILITE GCK 12 par la société Ajinomoto.
   - les citrates tels que le mono-ester citrique d'alcools de coco oxyéthylénés (9 moles), commercialisé sous la dénomination WITCONOL EC 1129 par la société Goldschmidt.
   - les galacturonates tels que le dodeécyl d-galactoside uronate de sodium commercialisé par la société Soliance.
5) Comme sulfosuccinates, on peut citer par exemple le mono-sulfosuccinate d'alcool laurylique (C₁₂/C₁₄ 70/30) oxyéthyléné (3 OE) commercialisé sous les dénominations SETACIN 103 SPECIAL^{®}, REWOPOL SB-FA 30 K 4^{®} par la société Witco, le sel di-sodique d'un hemi-sulfosuccinate des alcools C₁₂-C₁₄, commercialisé sous la dénomination SETACIN F SPECIAL PASTE^{®} par la société Zschimmer Schwarz, l'oléamidosulfosuccinate di-sodique oxyéthyléné (2 OE) commercialisé sous la dénomination STANDAPOL SH 135^{®} par la société Cognis, le mono-sulfosuccinate d'amide laurique oxyéthyléné (5 OE) commercialisé sous la dénomination LEBON A-5000^{®} par la société Sanyo, le sel di-sodique de mono-sulfosuccinate de lauryl citrate oxyéthyléné (10 OE) commercialisé sous la dénomination REWOPOL SB CS 50^{®} par la société Witco, le mono-sulfosuccinate de mono-éthanolamide ricinoléique commercialisé sous la dénomination REWODERM S 1333^{®} par la société Witco. On peut utiliser aussi les sulfosuccinates de polydimethylsiloxane tels que le disodium PEG-12 dimethicone sulfosuccinate commercialisé sous la dénomination MACKANATE-DC30 par la société Mac Intyre.
6) Comme alkyl sulfates, on peut citer par exemple le lauryl sulfate de triéthanolamine (nom INCI : TEA-lauryl sulfate) tel que le produit commercialisé par la société Huntsman sous la dénomination EMPICOL TL40 FL ou celui commercialisé par la société Cognis sur la dénomination TEXAPON T42, produits qui sont à 40 % en solution aqueuse. On peut aussi citer le lauryl sulfate d'ammonium (nom CFTA : Ammonium lauryl sulfate) tel que le produit commercialisé par la société Huntsman sous la dénomination EMPICOL AL 30FL qui est à 30 % en solution aqueuse.
7) Comme alkyl éther sulfates, on peut citer par exemple le lauryl éther sulfate de sodium (nom INCI : sodium laureth sulfate) comme celui commercialisé sous les dénominations TEXAPON N40 et TEXAPON AOS 225 UP par la société Cognis ou comme celui commercialisé sous la dénomination EMPICOL ESB 3/FL3 par la société Huntsman, le lauryl éther sulfate d'ammonium (nom INCI : ammonium laureth sulfate) comme celui commercialisé sous la dénomination STANDAPOL EA-2 par la société Cognis.
8) Comme sulfonates, on peut citer par exemple les alpha-oléfines sulfonates comme l'alpha-oléfine sulfonate de sodium (C₁₄₋₁₆) commercialisé sous la dénomination BIO-TERGE AS-40^{®} par la société Stepan, commercialisé sous les dénominations WITCONATE AOS PROTEGE^{®} et SULFRAMINE AOS PH 12^{®} par la société Witco ou commercialisé sous la dénomination BIO-TERGE AS-40 CG^{®} par la société Stepan, l'oléfine sulfonate de sodium secondaire commercialisé sous la dénomination HOSTAPUR SAS 30^{®} par la société Clariant ; les alkyl aryl sulfonates linéaires comme le xylène sulfonate de sodium commercialisé sous les dénominations MANROSOL SXS30^{®}, MANROSOL SXS40^{®}, MANROSOL SXS93^{®} par la société Manro.
9) Comme iséthionates, on peut citer les acyliséthionates comme le cocoyl-iséthionate de sodium, tel que le produit commercialisé sous la dénomination JORDAPON CI P^{®} par la société Jordan.
10) Comme taurates, on peut citer le sel de sodium de méthyltaurate d'huile de palmiste commercialisé sous la dénomination HOSTAPON CT PATE^{®} par la société Clariant ; les N-acyl N-méthyltaurates comme le N-cocoyl N-methyltaurate de sodium commercialisé sous la dénomination HOSTAPON LT-SF^{®} par la société Clariant ou commercialisé sous la dénomination NIKKOL CMT-30-T^{®} par la société Nikkol, le palmitoyl methyltaurate de sodium commercialisé sous la dénomination NIKKOL PMT^{®} par la société Nikkol.
11) Les dérivés anioniques d'alkyl-polyglucosides peuvent être notamment des citrates, tartrates, sulfosuccinates, carbonates et éthers de glycérol obtenus à partir des alkyl polyglucosides. On peut citer par exemple le sel de sodium d'ester tartrique de cocoylpolyglucoside (1,4), commercialisé sous la dénomination EUCAROL AGE-ET^{®} par la société Cesalpinia, le sel di-sodique d'ester sulfosuccinique de cocoylpolyglucoside (1,4), commercialisé sous la dénomination ESSAI 512 MP^{®} par la société Seppic, le sel de sodium d'ester citrique de cocoyl polyglucoside (1,4) commercialisé sous la dénomination EUCAROL AGE-EC^{®} par la société Cesalpinia.

### Tensioactifs moussants amphotères et zwitterioniques

Les tensioactifs amphotères et zwitterioniques peuvent être choisis par exemple parmi les bétaïnes, les N-alkylamidobétaïnes et leurs dérivés, les sultaïnes, les alkyl polyaminocarboxylates, les alkylamphoacétates et leurs mélanges.
1) Comme bétaïnes, on peut citer notamment les alkylbétaïnes comme par exemple la cocobétaïne comme le produit commercialisé sous la dénomination DEHYTON AB-30^{®} par la société Cognis, la laurylbétaïne comme le produit commercialisé sous la dénomination GENAGEN KB^{®} par la société Clariant, la laurylbétaïne oxyethylénée (10 OE), comme le produit commercialisé sous la dénomination LAURYLETHER (10 OE) BETAINE^{®} par la société Shin Nihon Rica, la stéarylbétaïne oxyéthylénée (10 OE) comme le produit commercialisé sous la dénomination STEARYLETHER (10 OE) BETAINE^{®} par la société Shin Nihon Rica.
   Parmi les N-alkylamidobétaines et leurs dérivés, on peut citer par exemple la cocamidopropyl bétaine commercialisée sous la dénomination LEBON 2000 HG^{®} par la société Sanyo, ou commercialisée sous la dénomination EMPIGEN BB^{®} par la société Albright & Wilson, la lauramidopropyl bétaïne commercialisée sous la dénomination REWOTERIC AMB12P^{®} par la société Witco.
2) Comme sultaines, on peut citer les hydroxylsultaïnes, telles que la cocamidopropyl hydroxysultaïne comme le produit commercialisé sous la dénomination REWOTERIC AM CAS par la société Golschmidt-Degussa, ou le produit commercialisé sous la dénomination CROSULTAINE C-50^{®} par la société Croda.
3) Comme alkyl polyaminocarboxylates (APAC), on peut citer le cocoylpolyamino-carboxylate de sodium, commercialisé sous la dénomination AMPHOLAK 7 CX/C^{®} et AMPHOLAK 7 CX^{®} par la société Akzo Nobel, le stéaryl-polyamidocarboxylate de sodium commercialisé sous la dénomination AMPHOLAK 7 TX/C par la société Akzo Nobel, la carboxyméthyloléyl-polypropylamine de sodium, commercialisé sous la dénomination AMPHOLAK XO7/C^{®} par la société Akzo Nobel.
4) Comme alkylamphoacétates, on peut citer par exemple le N-cocoyl-N-carboxyméthoxyéthyl-N-carboxyméthyl-éthylènediamine N-di-sodique (nom INCI: disodium cocoamphodiacetate) comme le produit commercialisé sous la dénomination MIRANOL C2M CONCENTRE NP^{®} par la société Rhodia, le N-cocoyl-N-hydroxyéthyl-N-carboxyméthyl-éthylènediamine N-sodique (nom INCI: sodium cocamphoacetate), le cocoamphohydroxypropyl sulfonate de sodium commercialisé sous la dénomination MIRANOL CSE par la société Rhodia.

Le gel moussant utilisé comme deuxième composition B selon l'invention comprend au moins 0,5 % en poids, et de préférence au moins 10 % en poids, de tensioactif(s) moussant(s) par rapport au poids total de la deuxième composition. Ainsi, les tensioactifs moussants (non ioniques, anioniques, amphotères et zwitterioniques) peuvent être présents en une quantité (en matière active) allant par exemple de 0,5 à 60 % en poids, notamment de 0,5 à 20 % en poids, de préférence de 1 à 15 % en poids et mieux de 2 à 10 % en poids par rapport au poids total de la seconde composition.

Ils peuvent plus particulièrement être présents en une quantité (en matière active) allant par exemple de 10 à 60% en poids, notamment de 15 à 60% en poids, par exemple de 20 à 60%, notamment de 25 à 55% en poids, et de préférence de 30 à 50% en poids par rapport au poids total de la seconde composition.

Quand la première composition A contient des tensioactifs moussants, ceux-ci peuvent être également dans les quantités indiquées ci-dessus,

Selon un mode particulier de réalisation de l'invention, la composition B contient au moins un tensioactif moussant non ionique, et comme tensioactif moussant autre que le tensioactif non ionique, au moins un tensioactif anionique et en particulier un dérivé anionique de protéines d'origine végétale ou de protéines de soie.

Selon un autre mode plus particulier de réalisation de l'invention, la composition B de l'invention contient au moins un alkylpolyglucoside et au moins un dérivé anionique de protéines d'origine végétale ou de protéines de soie.

### Viscosité des compositions formant un produit selon l'invention

Comme précisé précédemment, les deux compositions A et B formant le produit selon l'invention présentent avantageusement la même viscosité.

Ainsi, la viscosité des compositions de l'invention est préférentiellement supérieure à 2 Pa.s, plus particulièrement supérieure à 3 Pa.s. De préférence, elle est inférieure à 20 Pa.s notamment inférieure à 15 Pa.s.

A titre d'exemple, cette viscosité peut varier de 2 à 15 Pa.s, de préférence de 3 à 10 Pa.s et mieux de 3 à 7 Pa.s.

La viscosité des compositions est mesurée à la température ambiante (de 20 à 25 °C) et à pression ambiante, au Rhéomat 180 (mettler) avec un corps de mesure à 200 tours/min.

Cette viscosité est particulièrement intéressante en termes de préemption et manipulation du mélange des deux compositions.

Pour des raisons évidentes, cette viscosité peut nécessiter d'être ajustée au niveau de chacune des compositions formant le produit selon l'invention par le biais de composé(s) dédié(s) plus particulièrement à procurer un effet épaississant.

A titre illustratif de ces composés à caractère épaississant, on peut plus particulièrement citer les composés polymériques épaississants.

Pour des raisons évidentes, le choix de ces polymères est conditionné par la nature chimique de chacune des compositions concernées.

Ainsi, dans le cas de compositions à caractère huileux, à l'image des compositions A dite « premières compositions », l'utilisation de polymères anioniques tels que définis ci-après est privilégiée. En revanche, dans le cas des compositions B dites « deuxième composition » qui sont de nature aqueuse, s'avèrent plus particulièrement intéressants les polymères non ioniques oxyalkylénés et les polymères cationiques et amphotères tels que définis ci-après.

La première composition A peut comprendre de 0,5 à 4 % en poids, en particulier de 0,8 à 3 en poids de polymère(s) épaississant(s).

La deuxième composition B peut comprendre de 0,5 à 5 % en poids, en particulier de 2 à 4 % en poids de polymère(s) épaississant(s).

### Polymères non ioniques oxyalkylénés

Ce sont des composés comprenant au moins un groupe choisi parmi les groupes d'oxyde d'éthylène (OE), les groupes d'oxyde de propylène (OP) et leurs mélanges (OE/OP). Les composés peuvent donc être des composés oxyéthylénés, des composés oxypropylénés, ou des composés oxyéthylénés / oxypropylénés. Ces composés ne sont pas des tensioactifs mais ils ont des propriétés épaississantes et ont une plus grand nombre de motifs oxyéthylénés et/ou oxypropylénés, en particulier un nombre supérieur à 350.

Les composés oxyalkylénés non ioniques peuvent être choisis notamment parmi les polyéthylène glycols, les esters d'acide gras et de polyéthylène glycol et/ou de polypropylène glycol, les dérivés alkyl- ou acyl-alkoxylés et notamment de polyol, les triesters de glycérol et d'acides gras oxyalkylénés et notamment oxyéthylénés, les dérivés d'amides gras oxyéthylénés ou oxypropylénés, les dérivés uréthanes oxyéthylénés modifiés par des chaînes alkyle, et leurs mélanges.
1. Les polyéthylènes glycols pouvant être utilisés dans la composition de l'invention sont des polycondensats d'oxyde d'éthylène. De préférence, ces polyéthylènes glycols ont un nombre de motifs d'oxyde d'éthylène (OE) supérieur 1000. Le nombre d'oxyde d'éthylène peut aller par exemple de 1000 à 50000 et de préférence de 5000 à 10000. Comme polyéthylène glycols, on peut citer par exemple le polyéthylène glycol comportant 7000 OE (nom INCI : PEG-7M) comme le produit commercialisé sous la dénomination POLYOX WSR N-750^{®} par la société Amerchol, le polyéthylène glycol comportant 14000 OE (nom INCI : PEG-14M) comme le produit commercialisé sous la dénomination POLYOX WSR 205 par la société Amerchol, le polyéthylène glycol comportant 20000 OE (nom INCI : PEG-20M) comme le produit commercialisé sous la dénomination POLYOX WSR 1105^{®} par la société Amerchol.
2. Les esters d'acide gras et de polyéthylène glycol et/ou de polypropylène glycol sont des condensats de polyéthylène glycol et/ou polypropylène glycol avec un ou plusieurs acides gras. Ce sont des composés de formule (II) :

   RCOO - (OE)ₘ - (OP)ₙ - R' (II)

   dans laquelle 0<m<300 et 0<n<300 et m+n > 25, de préférence >50, R et R' représentent indépendamment l'un de l'autre l'hydrogène ou une chaîne alkyle saturée ou insaturée, linéaire ou ramifiée, hydroxylée ou non, comportant de 1 à 30 atomes de carbone et de préférence de 12 à 22 atomes de carbone, ou une chaîne aryle, à condition que R et R' ne soient pas en même temps l'hydrogène.
   Comme esters d'acide gras et de polyéthylène glycol et/ou de polypropylène glycol, on peut citer par exemple le distéarate de polyéthylène glycol (150 OE) tel que le produit commercialisé sous la dénomination ATLAS G-1821^{®} par la société Uniqema, le PEG-150 dibehenate tel que le produit commercialisé sous la dénomination ETHOX PEG 6000 Dibehenate^{®} par la société Ethox, le palmito- stéarate de polyéthylène glycol (120 OE) tel que le produit commercialisé sous la dénomination STEARATE 6000 WL 1644^{®} par la société Gattefosse, le copolymère de polyéthylène glycol (30 OE) et d'acide 12- hydroxystéarate tel que le produit commercialisé sous la dénomination ARLACEL P135^{®} par la société Uniqema, le stéarate de polyéthylène glycol (40 OE) tel que le produit commercialisé sous la dénomination MYRJ 52^{®} par la société Uniqema.
   Dans le cas où dans la formule (II), R=R'=H, on peut citer par exemple le copolymère statistique polyoxyéthylène / polyoxypropylène (17 OE / 6 OP) commercialisé sous la référence UCON 75-H-450^{®} par la Société Amerchol. Les molécules comportant plus d'OE et/ou plus d'OP ne sont pas exclues.
3. Les dérivés alkyl ou acyl alkoxylés de polyol peuvent être notamment des dérivés alkyl ou acyl éthoxylés de polyol, par exemple des dérivés oxyéthylénés d'esters d'acide gras et de polyol ou des dérivés oxyéthylénés d'éthers d'alcool gras et de polyol, et notamment des dérivés oxyéthylénés d'esters d'acide gras ou d'éthers d'alcool gras et de glycérol ou de sorbitol ou de glucose ou de pentaérythritol.
   Comme dérivés de ce type, on peut citer par exemple, le cocoate de glycéryle oxyéthyléné (78 OE) tel que le produit commercialisé sous la dénomination SIMULSOL CG par la société Seppic, le di-oléate de méthyl-glucose oxyéthyléné (120 OE) (nom INCI: PEG-120 méthylglucose dioléate) tel que le produit commercialisé sous la dénomination GLUCAMATE DOE-120 VEGETAL^{®} par la société Amerchol, le septa-oléate de sorbitane oxyéthyléné (40 OE) tel que le produit commercialisé sous la dénomination ARLATONE T^{®} par la société Uniqema, le laurate de polyglycéryle (2 moles de glycérol) oxyéthyléné (10 OE) tel que le produit commercialisé sous la dénomination HOE S 3495^{®} par la société Clariant, l'isostéarate de glycéryle oxyéthyléné (60 OE) tel que le produit commercialisé sous la dénomination EMALEX GWIS-160^{®} par la société SACI-CFPA, le monostéarate de glycéryle oxyéthyléné (20 OE) tel que le produit commercialisé sous la dénomination CUTINA E 24^{®} par la société Cognis, le stéarate de glycéryle oxyéthyléné (200 OE) tel que le produit commercialisé sous la dénomination SIMULSOL 220 TM^{®} par la société Seppic, le tétrastéarate de pentaérythrityle oxyéthyléné (150 OE) tel que le produit commercialisé sous la dénomination CROTHIX^{®} par la société Croda, le tristérate de sorbitan oxyéthyléné (160 OE) tel que le produit commercialisé sous la dénomination RHEODOL TW IS399C par la société.Kao Chemicals.
4. Comme triesters de glycérol et d'acides gras oxyalkylénés, on peut citer par exemple les glycérides d'acide caprylique/caprique oxyéthylénés (6 OE), tels que le produit commercialisé sous la dénomination SOFTIGEN 767^{®} par la société Condea, et l'huile d'olive oxyéthylénée (50 OE) tel que le produit commercialisé sous la dénomination CROVOL O-70^{®} par la société Croda.
5. Comme dérivés d'amides d'acide gras oxyalkylénés, on peut citer notamment les amides d'acide gras oxypropylénés comme par exemple le PPG-2 HYDROXYETHYL COCAMIDE et les mélanges en contenant tels que les produits commercialisés par Uniqema sous la dénomination Promidium, notamment Promidium CO.
6. Comme dérivés uréthanes oxyéthylénés modifiés par des chaînes alkyle, on peut citer par exemple ceux de formules (III) et (IV) :

   R₁NH-CO-(OCH₂CH₂)ₐ-[O-CO-NR₄-R₃-NR₄-CO-(OCH₂CH₂)ₐ]_{b}-O-CO-NH R₂ (III)

   R₅-(OCH₂CH₂)ₙ-O-CO-NH-R₆-NH-CO-(OCH₂CH₂)ₙ-OR₅ (IV)

   dans lesquelles les radicaux R₁, R₂ et R₅ représentent un groupe alkyle de C₁₋₁₈ ; R₃ et R₆ représentent un radical hydrocarboné linéaire, cyclique ou aromatique en C₄₋₃₆ ; R₄ représente un atome d'hydrogène ou un radical alkyle en C₁₋₆, de préférence un atome d'hydrogène ; a et n sont des nombres entiers allant de 90 à 600, et b est un nombre entier allant de 1 à 4.

Il s'agit par exemple de polymères hydrosolubles obtenus par réaction d'addition de diisocyanates (HMDI: Hexamethylene diisocyanate) sur des diols (polyéthers ou polyesters) et terminés par des groupements hydrophobes provenant d'alcools gras éthoxylés ou éthoxylés/propoxylés. C'est le cas par exemple du NUVIS FX 1100, commercialisé par la société Elementis, qui est un copolymère alcool stéarylique oxyéthyléné (100 OE) / polyéthylène glycol (136 OE) /hexaméthylène diisocyanate (nom INCI : steareth-100/PEG-136/HMDI copolymer).

Comme polymères non ioniques, on utilise de préférence ceux choisis parmi les dérivés alkyl ou acyl oxyéthylénés d'esters d'acide gras ou d'éthers d'alcool gras et de polyol, notamment des dérivés oxyéthylénés d'esters d'acide gras ou d'éthers d'alcool gras et de glycérol ou de sorbitol ou de glucose ou de pentaérythritol, plus particulièrement le di-oléate de méthyl-glucose oxyéthyléné (120 OE) (nom INCI : PEG-120 méthylglucose dioléate).

### Polymères cationiques et amphotères

Comme polymères cationiques ou amphotères, on peut citer notamment ceux du type Polyquaternium (nom INCI), qui apportent douceur et onctuosité à la crème moussante. Ces polymères peuvent être choisis de préférence parmi les polymères suivants :
- Polyquaternium 5 tel que le produit MERQUAT 5 commercialisé par la société CALGON ;
- Polyquaternium 6 tel que le produit SALCARE SC 30 commercialisé par la société CIBA, et le produit MERQUAT 100 commercialisé par la société CALGON ;
- Polyquaternium 7 tel que les produits MERQUAT S, MERQUAT 2200 et MERQUAT 550 commercialisés par la société CALGON, et le produit SALCARE SC 10 commercialisé par la société CIBA ;
- Polyquaternium 10 tel que le produit Polymer JR400 commercialisé par la société AMERCHOL ;
- Polyquaternium 11 tel que les produits GAFQUAT 755, GAFQUAT 755N et GAFQUAT 734 commercialisés par la société ISP ;
- Polyquaternium 15 tel que le produit ROHAGIT KF 720 F commercialisé par la société ROHM ;
- Polyquaternium 16 tel que les produits LUVIQUAT FC905, LUVIQUAT FC370, LUVIQUAT HM552 et LUVIQUAT FC550 commercialisés par la société BASF ;
- Polyquaternium 22 tel que le produit MERQUAT 280 commercialisé par la société CALGON ;
- Polyquaternium 28 tel que le produit STYLEZE CC10 commercialisé par la société ISP ;
- Polyquaternium 39 tel que le produit MERQUAT PLUS 3330 commercialisé par la société CALGON ;
- Polyquaternium 44 tel que le produit LUVIQUAT CARE commercialisé par la société BASF ;
- Polyquaternium 46 tel que le produit LUVIQUAT HOLD commercialisé par la société BASF ;
- Polyquaternium 47 tel que le produit MERQUAT 2001 commercialisé par la société CALGON.

On peut aussi utiliser comme polymère cationique les guars cationiques telles que les produits JAGUAR commercialisés par la société RHODIA.

Comme polymères cationiques, on utilise de préférence le Polyquaternium 7, le Polyquaternium 14 et Polyquaternium 47.

### Polymères anioniques

Comme polymères anioniques, on peut citer notamment ceux comportant au moins une chaîne hydrophobe, et en particulier ceux dérivés d'acide acrylique ou méthacrylique, comme le copolymère acrylates/steareth-20 methacrylate commercialisé sous la dénomination Aculyn 22 par la société Rohm & Haas (nom INCI : Acrylates/Steareth-30 Methacrylate copolymer) ; le terpolymère acide (méth)acrylique / acrylate d'éthyle / méthacrylate de béhényle oxyéthyléné (25 OE), en émulsion aqueuse commercialisé sous la dénomination Aculyn 28 par la société Rohm & Haas ; le copolymère acide acrylique/itaconate de mono-cétyle oxyéthyléné (20 OE), en dispersion aqueuse à 30 % commercialisé sous la dénomination Structure 3001 par la société National Starch ; le copolymère acide acrylique/itaconate de mono-stéaryle oxyéthyléné (20 OE) en dispersion aqueuse à 30 % commercialisé sous la dénomination Structure 2001 par la société National Starch ; le copolymère acrylates/acrylate modifié par des alcools en C₁₂-C₂₄ polyoxyéthylénés (25 OE), sous forme de latex à 30-32 % de copolymère, commercialisé sous la dénomination Synthalen W2000 par la société 3V SA, ainsi que les copolymères vendus sous les noms PEMULEN ou CARBOPOL par la Société Noveon, comme le copolymère acrylate/C₁₀-C₃₀-alkylacrylate tel que les produits PEMULEN TR1, PEMULEN TR2 ou CARBOPOL 1382 (nom INCI : Acrylates/C10-30 Alkyl acrylate Crosspolymer).

Comme polymères anioniques, on peut citer aussi les polymères et copolymères d'acide 2-acrylamido-2-méthylpropane sulfonique (AMPS) tels que :
- l'homopolymère réticulé et neutralisé d'acide 2-acrylamido 2-méthylpropane sulfonique, commercialisé par la société Clariant sous la dénomination commerciale « Hostacerin AMPS » (nom INCI : ammonium polyacryldimethyltauramide) ;
- les copolymères anioniques réticulés d'acrylamide ou méthylacrylamide et d'acide 2-acrylamido-2-méthylpropane sulfonique, notamment ceux se présentant sous la forme d'une émulsion E/H, tels que ceux commercialisés sous le nom de SEPIGEL 305 par la société Seppic (nom INCI : Polyacrylamide / C13-14 Isoparaffin / Laureth-7), sous le nom de SIMULGEL 600 par la société Seppic (nom INCI : Acrylamide / Sodium acryloyldimethyltaurate copolymer / Isohexadecane / Polysorbate 80),
- les copolymères d'acide(méth)acrylique ou de (méth)acrylate et d'acide 2-acrylamido- 2-méthylpropane sulfonique, notamment ceux se présentant sous la forme d'une émulsion E/H, tels que ceux commercialisés sous le nom de SIMULGEL NS par la société Seppic (copolymère d'acrylamido-2-methyl propane sulfonate de sodium / hydroxyethyl acrylate en émulsion inverse à 40% dans Polysorbate 60 et squalane) (nom INCI : hydroxyethyl acrylate/sodium acryloyldimethyltaurate copolymer / squalane / polysorbate 60) ou ceux commercialisés sous le nom de SIMULGEL EG par la société Seppic (copolymère d'acide acrylique / acrylamido-2-methylpropane-sulfonique sous forme de sel de sodium en émulsion inverse à 45% dans isohexadecane / eau) (nom INCI : Sodium Acrylate / Sodium acryloydimethyl-taurate copolymer / Isohexadecane / Polysorbate 80),
- les copolymères d'acide 2-acrylamido-2-méthylpropane sulfonique et de vinylpyrrolidone ou de vinylformamide, tels que les produits commercialisés sous les dénominations ARISTOFLEX AVC par la société Clariant,
- les polymères d'AMPS modifiés hydrophobes comme notamment le copolymère d'AMPS et de methacrylate d'alcool en C12-C14 éthoxylé (copolymère non réticulé obtenu à partir de Genapol LA-070 et d'AMPS) (nom INCI : Ammonium Acryloyldimethyltaurate / Laureth-7 Methacrylate Copolymer) commercialisé sous la dénomination ARISTOFLEX LNC par la société Clariant, et le copolymère d'AMPS et de méthacrylate de stéaryle éthoxylé (25 EO) (copolymère réticulé par le trimethylolpropane triacrylate, obtenu à partir de Genapol T-250 et d'AMPS) (nom INCI : Ammonium Acryloyldimethyltaurate / Steareth-25 Methacrylate Crosspolymer) commercialisé sous la dénomination ARISTOFLEX HMS par la société Clariant, et le copolymère d'AMPS et de méthacrylate d'alcool en C16-C18 comportant de 6 à 10 groupes oxyéthylénés, plus particulièrement le copolymère d'AMPS et d'acide méthacrylique ou d'un sel d'acide méthacrylique et d'alcool stéarylique oxyéthyléné comportant 8 moles d'oxyde d'éthylène (Genapol T-080) tel que celui commercialisé sous la dénomination Aristoflex SNC par la société Clariant (nom INCI: Ammonium Acryloyldimethyltaurate / Steareth-8 Methacrylate Copolymer).

On peut aussi utiliser dans la composition contenant de l'huile, des épaississants d'huiles, comme par exemple les argiles modifiées telles que le silicate de magnésium modifié (bentone gel VS38 de RHEOX), l'hectorite modifiée par le chlorure de distéaryl diméthyl ammonium (nom INCI : Disteardimonium hectorite) commercialisé sous la dénomination « bentone 38 CE » par la société RHEOX ; et comme les polymères semi-cristallins (homopolymères ou copolymères) tels que les homopolymères résultant de la polymérisation d'un monomère à chaîne cristallisable choisi parmi les acrylates d'alkyle en C₁₄-C₂₂ et les méthacrylates d'alkyle en C₁₄-C₂₂, tel que notamment ceux commercialisés sous les dénominations Intelimer^{®} par la société Landec, décrits dans la brochure "Intelimer^{®} polymers", Landec IP22 (Rev. 4-97). On peut citer plus particulièrement l'homopolymère d'acrylate de stéaryle (Intelimer IPA-13.1) (nom INCI : Poly C10-30 alkyl acrylate), l'homopolymère d'acrylate de béhényle (Intelimer IPA-13.6) (nom INCI : Poly C10-30 alkyl acrylate).

On peut aussi utiliser des copolymères d'acrylates d'alkyle en C₁₄-C₂₂ ou de méthacrylates d'alkyle en C₁₄-C₂₂ avec notamment l'acide acrylique. Comme copolymères, on peut citer les copolymères obtenus par la copolymérisation de l'acrylate de béhényle et de l'acide acrylique, ou les copolymères obtenus par la copolymérisation de l'acrylate de stéaryle et de l'acide acrylique.

Comme polymères anioniques, on utilise de préférence les polymères et copolymères d'AMPS, en particulier les copolymères anioniques réticulés d'acrylamide ou méthylacrylamide et d'acide 2-acrylamido-2-méthylpropane sulfonique, se présentant sous la forme d'une émulsion E/H, tels que le SIMULGEL 600 (nom INCI : Acrylamide / Sodium acryloyldimethyltaurate copolymer / Isohexadecane / Polysorbate 80).

### Milieu physiologiquement acceptable

Au sens de l'invention, un milieu physiologiquement acceptable désigne un milieu non toxique et susceptible d'être appliqué sur les matières kératiniques d'êtres humains, et d'aspect, d'odeur et de toucher agréables.

Comme précisé précédemment, la seconde composition contient au moins, à titre de milieu physiologiquement acceptable, de l'eau ou une phase aqueuse.

Une phase aqueuse contient de l'eau en mélange avec un ou plusieurs composés hydrosolubles.

Dans le cas où la première composition se présente sous la forme d'une émulsion, celle-ci contient également de l'eau ou une phase aqueuse.

L'eau représente préférentiellement de 40 à 82% en poids, notamment de 40 à 80 % en poids, par exemple de 45 à 75% en poids, en particulier de 50 à 70% en poids par rapport au poids total de la seconde composition B.

A l'inverse, l'eau ou la phase aqueuse représente préférentiellement moins de 40 % en poids, en particulier moins de 30 % en poids, mieux moins de 25% en poids par exemple moins de 20% en poids, et de préférence moins de 15% en poids par rapport au poids total de la première composition A.

Généralement, on entend par « eau » de l'eau pure ou déminéralisée.

Toutefois, une partie de l'eau utilisée dans les compositions de l'invention peut éventuellement être choisie parmi les eaux minérales ou thermales.

Par « eaux minérales ou thermales », on désigne non seulement les eaux minérales ou thermales naturelles, mais également des eaux minérales ou thermales naturelles enrichies en constituants minéraux et/ou en oligo-éléments supplémentaires, ainsi que des solutions aqueuses minérales et/ou oligo-élémentaires préparées à partir d'eau purifiée, déminéralisée ou distillée.

Une eau thermale ou minérale naturelle utilisée selon l'invention peut, par exemple être choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevard-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-Bains, l'eau d'Uriagle-les-bains, l'eau d'Avene.

Les milieux physiologiquement acceptables formant les compositions de l'invention peuvent, en outre, contenir des adjuvants habituels dans le domaine cosmétique ou dermatologique, tels que les gélifiants hydrophiles ou lipophiles, les actifs hydrophiles ou lipophiles, les conservateurs (par exemple phenoxyethanol et parabens), les antioxydants, les solvants, les parfums, les charges, les filtres UV, les bactéricides, les absorbeurs d'odeur, les matières colorantes, les sels.

Les quantités de ces différents adjuvants sont celles classiquement utilisées dans le domaine considéré, et par exemple de 0,01 à 20 % du poids total de la composition les contenant. Ces adjuvants, selon leur nature, peuvent être introduits dans l'une ou l'autre des compositions formant le produit selon l'invention.

Comme charges qui peuvent être utilisées dans l'une et/ou l'autre des compositions de l'invention, on peut citer par exemple, outre les pigments, la poudre de silice ; le talc ; les particules de polyamide et notamment celles vendues sous la dénomination ORGASOL par la société Atochem ; les poudres de polyéthylène ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les micro-sphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélanges.

Ces charges peuvent être présentes dans des quantités allant de 0 à 20 % en poids et de préférence de 1 à 10 % en poids par rapport au poids total de la composition les contenant.

Selon un mode de réalisation particulièrement avantageux, les produits selon l'invention peuvent comprendre en outre des agents exfoliants.

Comme agents exfoliants, on peut citer par exemple des particules exfoliantes ou gommantes d'origine minérale, végétale ou organique. Ainsi, on peut utiliser par exemple des billes ou de la poudre de polyéthylène, de la poudre de nylon, de la poudre de polychlorure de vinyle, de la pierre ponce, des broyats de noyaux d'abricots ou de coques de noix, de la sciure de bois, des billes de verre, l'alumine et leurs mélanges.

Ces particules peuvent être présentes en une quantité allant par exemple de 0,5 à 40 % en poids, de préférence de 1 à 20 % en poids et mieux de 1 à 10 % en poids par rapport au poids total de la composition les contenant.

Quand au moins l'une des compositions formant le produit selon l'invention contient des particules exfoliantes, ce dernier peut constituer notamment un produit de gommage de la peau du visage ou du corps.

Comme actifs utilisables dans les produits de l'invention, on peut citer par exemple, les vitamines hydrosolubles ou liposolubles comme la vitamine A (rétinol), la vitamine E (tocophérol), la vitamine C (acide ascorbique) et ses dérivés tels que l'ascorbyl glucoside, la vitamine B5 (panthénol), la vitamine B3 (niacinamide), les dérivés de ces vitamines (notamment esters) et leurs mélanges ; les antiseptiques ; les actifs anti-bactériens comme le 2,4,4'-trichloro-2'-hydroxy diphényl éther (ou triclosan), le 3,4,4'-trichlorocarbanilide (ou triclocarban) ; les anti séborrhéiques ; les antimicrobiens tels que le peroxyde de benzoyle, l'acide salicylique, l'acide azélaïque, la niacine (vit. PP) ; les amincissants tels que la caféine ; les azurants optiques, et tout actif approprié pour le but final de la composition, et leurs mélanges.

Les choix de ces actifs est ajusté en fonction de la qualité retenue pour le produit selon l'invention, par exemple l'acide salicylique, l'acide azélaïque, le triclosan, le piroctone olamine, la niacinamide (vitamine PP) pour le traitement des peaux grasses.

### Ensemble de conditionnement

Comme précisé précédemment, l'invention vise également un ensemble de conditionnement.

Plus particulièrement, cet ensemble comporte au moins :
i. un premier compartiment contenant une première composition A se présentant sous la forme d'une émulsion huile-dans-eau obtenue selon le procédé d'inversion de phase en température selon la technologie PIT et comprenant dans un milieu physiologiquement acceptable, au moins 50 % en poids d'au moins une huile par rapport au poids total de ladite composition,
ii. un second compartiment contenant une seconde composition se présentant sous la forme d'un gel aqueux et contenant au moins un tensioactif moussant, ledit second compartiment étant isolé de manière étanche du premier, et
iii. des moyens pour permettre la mise en contact notamment extemporanée des deux compositions.

Un tel ensemble permet avantageusement la mise en contact extemporanée de ses deux compositions, conditionnées séparément dans respectivement les premier et second compartiments formant ledit ensemble.

Cet ensemble peut être également muni de moyens permettant la mise en communication des premier et second compartiments et donc de leurs contenus respectifs.

L'ensemble est également avantageusement muni d'un moyen propice à la distribution du mélange des deux compositions.

Plus précisément, les compositions A et B utilisées pour la mise en oeuvre de l'invention sont conditionnées séparément à l'intérieur de deux compartiments, formés soit par deux récipients distincts, soit à l'intérieur d'un dispositif unitaire.

Par « dispositif unitaire » on entend un dispositif par lequel les deux compartiments sont solidaires l'un de l'autre. Un tel dispositif peut être obtenu par un procédé de moulage en une seule pièce des deux compartiments, notamment en un matériau thermoplastique. Il peut également résulter de toute forme d'assemblage, notamment par collage, soudage, ou autre encliquetage.

Selon un premier mode de réalisation, les deux récipients sont indépendants l'un de l'autre. De tels récipients peuvent se présenter sous diverses formes. Il peut s'agir notamment de tubes, de flacons ou de bidons.

L'un et/ou l'autre des récipients peuvent être surmontés d'une pompe à actionnement manuel surmontée d'un bouton poussoir pour l'actionnement de la pompe et la distribution de la composition *via* au moins un orifice de distribution.

Alternativement, l'un et/ou l'autre des récipients sont pressurisés, notamment au moyen d'un agent propulseur, en particulier un gaz propulseur. Dans ce cas, le (ou les) récipient(s) est (sont) équipé(s) d'une valve surmontée par un bouton poussoir équipée d'une buse ou de tout autre moyen de diffusion pour la distribution du produit.

Le propulseur peut être en mélange avec la composition à distribuer ou séparé, notamment *via* un piston apte à coulisser à l'intérieur du récipient, ou *via* les parois souples d'une poche à l'intérieur de laquelle est disposée la composition.

Les récipients peuvent être constitués de matériaux divers : plastique, verre, ou métal.

Selon un mode de réalisation préféré, les deux compositions sont contenues à l'intérieur d'un dispositif unitaire.

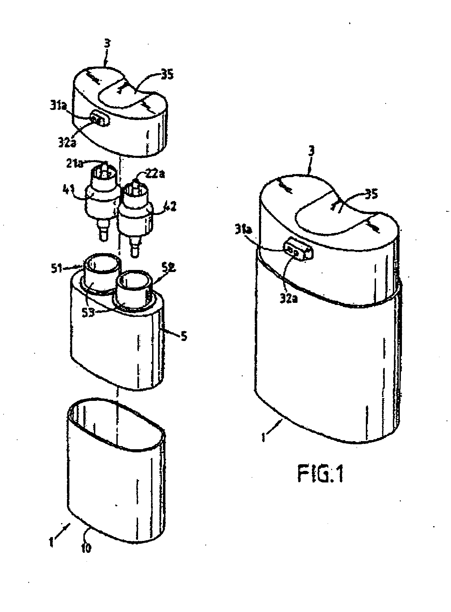

Selon le mode de réalisation représenté à la figure 1, le dispositif de conditionnement 1 est constitué de deux compartiments 51, 52 disposés côte à côte et formés à l'intérieur d'une pièce 5 obtenue de moulage d'un matériau thermoplastique. Chacun des récipients 51, 52 comporte un col 53 délimitant une ouverture. A l'intérieur du col de chacun des récipients est montée une pompe 41, 42, qui peut être avec ou sans reprise d'air.

Lors du montage, la pièce 5 délimitant les deux compartiments 51, 52, est disposée à l'intérieur d'un élément d'habillage 10.

Une tige de pompe 21a, 22a, de chacune des pompes 41, 42 est insérée à force à l'intérieur d'un conduit correspondant prévu dans un bouton poussoir unique 3 configuré de manière à permettre l'actionnement simultané des deux pompes, en réponse à une pression exercée axialement sur une surface 35 du bouton poussoir 3.

Les conduits du bouton poussoir connectés à chacune des pompes débouchent sur deux orifices 31a, 32a, disposés au voisinage l'un de l'autre sur une surface extérieure du bouton poussoir 3. En réponse à un actionnement des pompes 41, 42, les deux compositions sortent séparément soit sur le doigt de l'utilisatrice, soit sur un tampon ou coton applicateur. Le mélange des deux compositions se fait alors lors de l'application sur la surface à traiter.

Selon un autre mode de réalisation non illustré, les deux récipients sont pressurisés et équipés d'une valve à enfoncement ou basculement. Les deux valves sont de préférence actionnables par un même bouton poussoir du type de celui décrit en référence au mode de réalisation à pompes.

Alternativement encore, les deux compartiments sont formés de deux compartiments concentriques formés à l'intérieur d'un tube, et le cas échéant sont surmontés d'une pompe sans reprise d'air équipée d'un bouton poussoir à un ou deux orifices de distribution. A l'intérieur du tube peut être prévu un piston qui remonte en direction de la pompe au fur et à mesure que les compositions sont prélevées à l'intérieur des récipients. De tels modes de distribution sont utilisés notamment pour la distribution de pâtes dentifrices.

D'autres dispositifs encore peuvent être utilisés pour la mise en oeuvre de la présente invention, l'essentiel étant qu'ils puissent permettre le conditionnement séparé des deux compositions et leur distribution de manière séparée ou en mélange.

A titre d'exemple encore les deux compositions sont conditionnées à l'intérieur de deux compartiments formés à l'intérieur d'un même sachet souple, les deux compartiments étant séparés par une zone de rupture qui peut être cassée au moment de l'utilisation, notamment en réponse à une pression exercée à un endroit précis du sachet.

L'invention concerne donc en particulier un ensemble, dans lequel les compositions A et B sont conditionnées à l'intérieur de deux compartiments formés par deux récipients distincts.

Selon un mode particulier, les compositions A et B sont conditionnées à l'intérieur de deux compartiments (51, 52) délimités par un dispositif unitaire (1) tel que représenté à la figure 1.

En particulier, chacun des compartiments est équipé d'une pompe (41, 42), de préférence à actionnement manuel, reliée à au moins un moyen d'actionnement et de distribution (3) permettant de délivrer, séparément ou en mélange les compositions A et B.

Selon un mode préféré, le moyen d'actionnement et de distribution (3) est commun aux deux pompes.

Selon une alternative, chacun des compartiments est pressurisé, notamment au moyen d'un agent propulseur, et équipé d'une valve reliée à au moins un moyen d'actionnement et de distribution permettant de délivrer, séparément ou en mélange les compositions A et B.

En particulier, le moyen d'actionnement et de distribution est commun aux deux valves.

On peut utiliser par exemple des dispositifs tels que décrits dans les documents US 5 833 121, US 4 773 562 et US 6 672 483.

Un ensemble ou un produit selon l'invention est plus particulièrement destiné à une application cosmétique de type nettoyage et/ou de démaquillage.

Les produits selon l'invention peuvent constituer notamment des produits de nettoyage ou de démaquillage de la peau (corps, visage, yeux), du cuir chevelu et/ou des cheveux.

Un autre objet de l'invention consiste en l'utilisation cosmétique d'un produit tel que défini ci-dessus, comme produit de nettoyage et/ou de démaquillage de la peau, du cuir chevelu et/ou des cheveux.

Les produits selon l'invention peuvent constituer conjointement un produit pour traiter les peaux grasses et/ou désinfecter la peau et/ou le cuir chevelu, notamment quand ils contiennent un antibactérien. En particulier, les actifs spécifiques de traitement des peaux grasses peuvent y être inclus, comme par exemple l'acide salicylique, l'acide azélaïque, le triclosan, le piroctone olamine, la niacinamide (vitamine PP).

Un autre objet de l'invention consiste en un procédé cosmétique de nettoyage de la peau, des yeux, du cuir chevelu et/ou des cheveux, caractérisé par le fait qu'on applique un mélange des compositions formant le produit selon l'invention, sur la peau, sur les yeux, sur le cuir chevelu et/ou sur les cheveux et qu'on élimine la mousse formée et les résidus de salissure par rinçage à l'eau.

Dans le cas du nettoyage du visage, le produit selon l'invention peut former un masque qui est rincé après un temps de pose de 1 à 3 minutes.

Les exemples qui suivent servent à illustrer l'invention sans toutefois présenter un caractère limitatif Les quantités indiquées sont en % en poids de matière première (et non de matière active) sauf mention contraire.

### Exemple 1

### Première composition contenant au moins 50 % d'huile

- Acide citrique 0,30 %
- Silice (Aerosol 200^{®} de la société Degussa-Hüls) 2,00 %
- Palmitate d'éthyl hexyle 50,00 %
- Colorants qs
- Conservateurs 0,80 %
- Parfums 0,40 %
- Acrylamide/Sodium Acryloyldimethyltaurate Copolymer (And) Isohexadecane (And) Polysorbate 80
   (Simulgel 600 de la société Seppic) 0,80 %
- Polyquaternium 47 (Merquat 2001 de la société Nalco à 20 % en matière active) 0,40 %
- Glycérine 6,50 %
- Cocobétaïne (vendue sous la référence DEHYTON AB 30 par la société Cognis à 100 % en matière active) 9,00 %
- Beheneth-10 (EUMULGIN BA10 de la société Cognis à 100 % en matière active) 4,00 %
- Décylglucoside (vendu sous la référence Mydol 10 par la société KAO à 40 % en matière active) 12,00 %
- Eau qsp 100,00 %

Mode opératoire : La phase contenant le beheneth-10, la glycérine, les conservateurs, les huiles et l'eau est chauffée à 85 °C sous Rayneri jusqu'à solubilisation des conservateurs. Le mélange ainsi obtenu devient alors opaque. Cette phase est alors refroidie à 60 °C. Alors, le mélange s'épaissit. On introduit alors la silice et le mélange se fluidifie. Après avoir homogénéisé quelques minutes, on refroidit à 45 °C. On introduit alors les tensioactifs moussants (Cocobétaïne, decylglucoside), puis, à température ambiante, on introduit l'acide citrique, les colorants, les parfums et les polymères.

### Exemple 2

### Première composition contenant au moins 50 % d'huile

- Silice (Aerosol 200^{®} de la société Degussa-Hüls) 1,00 %
- Palmitate d'éthylhexyle 68,00 %
- Conservateurs 0,40 %
- Ethanol 2,00 %
- Glycérine 10,00 %
- Beheneth-10 (EUMULGIN BA 10 de la société Cognis à 100 % en matière active) 5,00 %
- Eau qsp 100,0 %

Mode opératoire : Une phase contenant le beheneth-10, la glycérine, les conservateurs, les huiles et l'eau est tout d'abord chauffée à 85 °C sous Rayneri jusqu'à solubilisation des conservateurs. Le mélange ainsi obtenu devient alors opaque. Cette phase est alors refroidie à 60 °C. Le mélange épaissit. On introduit alors la silice et le mélange se fluidifie. Après avoir homogénéisé quelques minutes, on refroidit à 45 °C. On introduit alors les tensioactifs, puis, à température ambiante, on introduit les colorants, les parfums et les polymères.

### Exemple 3

### Deuxième composition de type gel aqueux

- Disodium EDTA 0,20 %
- Triéthanolamine 0,60 %
- Colorants qs
- Acide salicylique 0,60 %
- Conservateurs 0,50 %
- Parfums 0,50 %
- Copolymère acrylates/méthacrylate de Steareth-20 (ACULYN 22 POLYMER^{®} de la société Rohm & Haas) 3,00 %
- Butylène glycol 3,00 %
- Lauryl sulfate de triéthanolamine (TEXAPON T 42^{®} de la société Cognis à 40 % en matière active) 5,00 %
- Décylglucoside (MYDOL 10^{®} de la société KAO à 40 % en matière active) 2,00 %
- Sodium Lauroyl Oat Amino Acids (PROTEOL OAT^{®} de la société Seppic à 30 % en matière active) 4,00 %
- Eau qsp 100,0 %

Mode opératoire : L'acide salicylique, le butylène glycol et l'eau sont tout d'abord mélangés en chauffant jusqu'à solubilisation. Lorsque la température est redescendue à température ambiante, les autres composés sont ajoutés en introduisant en dernier les parfums, les tensioactifs et les polymères.

### Exemple 4

### Deuxième composition de type gel aqueux

- Citrate de sodium 0,04 %
- Sorbitol 3,50 %
- Conservateurs 0,45 %
- Parfums 0,20 %
- Glycérine 3,50 %
- Cocobétaïne (DEHYTON AB 30® de la société Cognis à 100 % en matière active) 32,50 %
- PEG-120 Methyl Glucose Dioleate (Glucamate DOE 120^{®} de la société Amerchol) 3,00 %
- Sodium laureth sulfate (EMPICOL ESB 3/FL3^{®} de la société Huntsman à 70 % en matière active) 3,70 %
- Glucoside d'ascorbyle 0,05 %
- Eau qsp 100,00 %

Mode opératoire : Le PEG-120 Methyl Glucose Dioleate, l'eau, la glycérine, et les conservateurs sont tout d'abord mélangés en chauffant jusqu'à solubilisation. Lorsque la température est redescendue à température ambiante, les autres composés sont ajoutés en introduisant en dernier le glucoside d'ascorbyle et le citrate de sodium.

### Exemple 5

### Deuxième composition de type gel aqueux

- Hydroxyde de potassium 4,00 %
- Disodium EDTA 0,20 %
- Sorbitol 3,50 %
- Colorants qs
- Acide salicylique 0,40 %
- Conservateurs 0,70 %
- Parfums 0,20 %
- Glycérine 3,50 %
- Pentylène Glycol 0,10 %
- PEG-150 Distéarate (Polyéthylèneglycol 6000 Distéarate^{®} de la société Akzo Nobel à 100 % en matière active) 1,00 %
- Decylglucoside (Mydol 10^{®} de la société KAO à 40 % en matière active) 16,25 %
- PEG-150 Pentaerythrityl Tétrastéarate (Crothix^{®} de la société Croda à 100 % en matière active) 1,00 %
- Sodium Cocoyl Glycinate (Amilite GCS 12 de la société d'Ajinomoto 30 % en matière active) 5,85 %
- Lauryl Phosphate (MAP 20 de la société Kao à 100 % en matière active) 6,50 %
- PEG-200 Glycéryl Stéarate (Simulsol 220 TM de la société Seppic) 2,00 %
- Eau qsp 100,00 %

Mode opératoire : Les gélifiants (PEG-150 Distéarate, PEG-150 Pentaerythrityl Tétrastéarate, PEG-200 Glycéryl Stéarate) l'eau, la glycérine, les conservateurs, le disodium EDTA, le sorbitol, les colorants et le pentylène glycol sont tout d'abord mélangés en chauffant jusqu'à solubilisation. L'hydroxyde de potassium et le laurylphosphate sont alors ajoutés, puis les autres tensioactifs. Lorsque la température est redescendue à température ambiante, les autres composés sont ajoutés en introduisant en dernier les parfums.

### Exemple 6 d'un produit conforme à l'invention

La première composition de l'exemple 2 et la même quantité de la deuxième composition de l'exemple 3 sont conditionnées dans deux compartiments distincts d'un conditionnement à double compartiments de type tube.

Les deux compositions sont mélangées en sortie de tube dans un rapport pondéral 50/50 avec de l'eau.

Le mélange correspondant est appliqué sur la peau d'un visage maquillé d'un film de maquillage de type fond de teint non transfert. Le démaquillage est réalisé efficacement et ne procure aucune sensation de dessèchement. A titre comparatif, cette même opération de démaquillage est reproduite uniquement avec la composition de l'exemple 1. Le démaquillage ainsi obtenu s'avère moins performant que celui obtenu avec le produit selon l'invention.

### Exemple 7 : comparatif

Un produit conforme à l'invention a été préparé en conditionnant dans deux compartiments distincts d'un ensemble de conditionnement de type tube, la première composition de l'exemple 2 et la même quantité de la deuxième composition de l'exemple 4.

A titre comparatif, un produit comprenant une première composition A' telle que décrite ci-après et une même quantité de ladite deuxième composition de l'exemple 4 a été préparé, de façon analogue.

La composition A' est la suivante :
- Silice (Aerosol 200^{®} de la société Degussa-Hüls) 1,00 %
- Palmitate d'éthylhexyle 68,00 %
- Conservateurs 0,40 %
- Ethanol 2,00 %
- Glycérine 10,00 %
- Mélange de mono/distéarate de glycéryle et de stéarate de PEG-100 (ARLACEL 165FI de CRODA) 5,00 %
- Eau qsp 100,0 %

Mode opératoire : L'Arlacel est mélangé dans l'huile et l'émulsion est réalisée à chaud à 65°C en mélangeant la phase aqueuse, comprenant la glycérine, les conservateurs, la silice, l'éthanol et l'eau, et la phase huileuse.

Il est tout d'abord constaté que la première composition A' ne mousse presque pas en comparaison avec la première composition de l'exemple 2.

Par ailleurs, il est également constaté que la première composition A' ne se mélange que faiblement avec la deuxième composition, contrairement à la première composition du produit conforme à l'invention.

Les performances de ces deux produits ont ensuite été comparées comme suit.

Pour chacun des produits, les deux compositions ont été mélangées en sortie de tube dans un rapport pondéral 50/50 avec de l'eau.

Le mélange correspondant a été appliqué sur la peau d'un visage maquillé d'un film de maquillage de type fond de teint non transfert.

Le démaquillage obtenu en mettant en oeuvre le produit comparatif s'avère moins performant que celui obtenu avec le produit conforme à l'invention.

Les performances des produits ont également été testées pour évaluer les propriétés démaquillantes des produits sur un rouge à lèvres longue tenue et sur un mascara waterproof.

On constate que le produit comparatif démaquille très peu comparativement au produit conforme à l'invention.

A la différence du produit comparatif, le produit conforme à l'invention mousse abondamment et immédiatement et permet d'enlever facilement et rapidement les films de mascara et de rouge à lèvres.

## Revendications

1. Produit cosmétique de nettoyage et/ou de démaquillage de matière(s) kératinique(s) comprenant, de manière séparée l'une de l'autre, au moins une première et une deuxième compositions cosmétiques, et dans lequel :
- la première composition A se présente sous la forme d'une émulsion huile-dans-eau obtenue selon le procédé d'inversion de phase en température selon la technologie PIT et comprend, dans un milieu physiologiquement acceptable, au moins 50 % en poids d'au moins une huile ou une phase huileuse, par rapport au poids total de la première composition, et
- la deuxième composition B se présente sous la forme d'un gel aqueux et comprend, dans un milieu physiologiquement acceptable, au moins un tensioactif moussant.

2. Produit selon la revendication précédente, dans lequel la première composition A comprend moins de 40 % en poids d'eau ou de phase aqueuse par rapport au poids total de la première composition A, en particulier moins de 30 % en poids, de préférence moins de 25 % en poids.

3. Produit selon l'une quelconque des revendications précédentes, dans lequel la première composition A comprend au moins un émulsionnant.

4. Produit selon la revendication précédente, dans lequel ledit émulsionnant est choisi parmi les alcools gras éthoxylés ou les acides gras éthoxylés ayant les formules (I) et (II) suivantes :
R-O-(CH₂-CH₂-O)ₘH (I)
R-COO(CH₂-CH₂-O)ₘH (II)
où R est une chaîne hydrocarbonée linéaire ou ramifiée, saturée ou insaturée, ayant de 10 à 24 atomes de carbone, et m est un nombre entier allant de 8 à 50.

5. Produit selon l'une quelconque des revendications précédentes, dans lequel la deuxième composition B comprend au moins 0,5 % en poids de tensioactif(s) moussant(s) par rapport au poids total de la deuxième composition, par exemple de 0,5 % à 60 % en poids, notamment de 10 à 60% en poids.

6. Produit selon l'une quelconque des revendications précédentes, dans lequel la deuxième composition B se présente sous la forme d'un gel aqueux transparent.

7. Produit selon l'une quelconque des revendications précédentes, dans lequel la deuxième composition B comprend en outre au moins un polymère choisi parmi les polymères non ioniques oxyalkylénés, les polymères cationiques et les polymères amphotères.

8. Produit selon l'une quelconque des revendications précédentes dans lequel la première et/ou la deuxième compositions présentent une viscosité supérieure à 2 Pa.s, à température ambiante de 20 à 25°C et à pression ambiante.

9. Produit selon l'une quelconque des revendications précédentes, **caractérisé en ce qu'**il constitue un produit nettoyant, démaquillant, un produit de gommage, un produit exfoliant.

10. Ensemble de conditionnement et de distribution des compositions formant un produit selon l'une quelconque des revendications 1 à 9, ledit ensemble comprenant au moins deux compartiments indépendants comprenant respectivement chacune desdites compositions et étant ajusté à la distribution des deux compositions de manière séparée ou en mélange.

11. Procédé de nettoyage et/ou démaquillage de matière(s) kératinique(s) comprenant au moins les étapes consistant à :
a) disposer d'une première composition A se présentant sous la forme d'une émulsion huile-dans-eau obtenue selon le procédé d'inversion de phase en température selon la technologie PIT et comprenant, dans un milieu physiologiquement acceptable, au moins 50 % en poids d'au moins une huile ou phase huileuse par rapport au poids total de ladite première composition,
b) disposer d'une seconde composition B se présentant sous la forme d'un gel aqueux et comprenant, dans un milieu physiologiquement acceptable, au moins un tensioactif moussant,
c) procéder à la mise en contact extemporanée de ladite première composition A avec ladite seconde composition B, et
d) appliquer le mélange obtenu à l'étape précédente sur ladite matière kératinique.

12. Procédé selon la revendication précédente dans lequel les première et seconde compositions sont telles que définies en revendications 1 à 9.

13. Procédé selon l'une quelconque des revendications 11 ou 12, dans lequel les étapes c) et d) sont réalisées simultanément.

14. Procédé selon l'une quelconque des revendications 11 à 13 dans lequel l'étape d) est réalisée en présence d'eau.

15. Procédé selon l'une quelconque des revendications 11 à 14 dans lequel l'étape d) est suivie d'une étape de rinçage avec de l'eau de la matière kératinique traitée.

## Claims

1. A cosmetic product for cleansing and/or removing makeup from keratinous substance(s) comprising, separately from one another, at least a first and a second cosmetic compositions, and in which:
- the first composition A is in the form of an oil-in-water emulsion obtained according to the phase inversion temperature process according to PIT technology and comprises, in a physiologically acceptable medium, at least 50% by weight of at least one oil or oily phase, relative to the total weight of the first composition; and
- the second composition B is in the form of an aqueous gel and comprises, in a physiologically acceptable medium, at least one foaming surfactant.

2. The product according to the preceding claim, in which the first composition A comprises less than 40% by weight of water or of aqueous phase relative to the total weight of the first composition A, in particular less than 30% by weight, preferably less than 25% by weight.

3. The product according to anyone of the preceding claims, in which the first composition A comprises at least one emulsifier.

4. The product according to the preceding claim, in which said emulsifier is chosen from ethoxylated fatty alcohols or ethoxylated fatty acids having the formulae (I) and (II) below:
R-O-(CH₂-CH₂-O)ₘH (I)
R-CO(CH₂-CH₂-O)ₘH (II)
where R is a saturated or unsaturated, linear or branched hydrocarbon-based chain having from 10 to 24 carbon atoms, and m is an integer ranging from 8 to 50.

5. The product according to anyone of the preceding claims, in which the second composition B comprises at least 0.5% by weight of foaming surfactant(s) relative to the total weight of the second composition, for example from 0.5% to 60% by weight, especially from 10% to 60% by weight.

6. The product according to anyone of the preceding claims, in which the second composition B is in the form of a transparent aqueous gel.

7. The product according to anyone of the preceding claims, in which the second composition B additionally comprises at least one polymer chosen among oxyalkylenated non ionic polymers, cationic polymers and amphoteric polymers.

8. The product according to anyone of the preceding claims, in which the first and/or the second compositions have a viscosity greater than 2 Pa.s at ambient temperature from 20 to 25°C and at ambient pressure.

9. The product according to anyone of the preceding claims, **characterized in that** it forms a cleansing, makeup-removing product, a scrubbing product or an exfoliating product.

10. A unit for packaging and dispensing compositions that form a product according to anyone of claims 1 to 9, said unit comprising at least two independent compartments that respectively comprise each of said compositions and that are adjusted for dispensing the two compositions separately or as a mixture.

11. A method for cleansing and/or removing makeup from keratinous substance(s) comprising at least the steps consisting in:
a) having a first composition A that is in the form of an oil-in-water emulsion obtained according to the phase inversion temperature process according to PIT technology and that comprises, in a physiologically acceptable medium, at least 50% by weight of at least one oil or oily phase relative to the total weight of said first composition;
b) having a second composition B that is in the form of an aqueous gel and that comprises, in a physiologically acceptable medium, at least one foaming surfactant;
c) bringing said first composition A into contact extemporaneously with said second composition B; and
d) applying the mixture obtained in the preceding step to said keratinous substance.

12. The method as claimed in the preceding claim, in which the first and second compositions are such as defined in claims 1 to 9.

13. The method as claimed in anyone of claims 11 or 12, in which the steps c) and d) are carried out simultaneously.

14. The method as claimed in anyone of claims 11 to 13, in which step d) is carried out in the presence of water.

15. The method as claimed in anyone of claims 11 to 14, in which step d) is followed by a step of rinsing the keratinous substance treated with water.

## Patentansprüche

1. Kosmetisches Reinigungs- und/oder Abschminkprodukt für keratinische Substanz(en), umfassend, getrennt voneinander, mindestens eine erste und eine zweite kosmetische Zusammensetzung, und wobei:
- die erste Zusammensetzung A in der Form einer Öl-in-Wasser-Emulsion vorliegt, die durch das Phaseninversions-Temperaturverfahren gemäß der PIT-Technologie erhalten wird, und, in einem physiologisch verträglichen Medium, mindestens 50 Gew.-% von mindestens einem Öl oder einer öligen Phase, bezogen auf das Gewicht der ersten Zusammensetzung, umfasst, und
- die zweite Zusammensetzung B in der Form eines wässrigen Gels vorliegt und, in einem physiologisch verträglichen Medium, mindestens ein schäumendes grenzflächenaktives Mittel umfasst.

2. Produkt nach dem vorhergehenden Anspruch, wobei die erste Zusammensetzung A weniger als 40 Gew.-% Wasser oder wässrige Phase, bezogen auf das Gesamtgewicht der ersten Zusammensetzung, insbesondere weniger als 30 Gew.-%, vorzugsweise weniger als 25 Gew.-% umfasst.

3. Produkt nach einem der vorhergehenden Ansprüche, wobei die erste Zusammensetzung A mindestens ein Emulgierungsmittel umfasst.

4. Produkt nach dem vorhergehenden Anspruch, wobei das Emulgierungsmittel aus ethoxylierten Fettalkoholen oder ethoxylierten Fettsäuren mit den folgenden Formeln (I) und (II):
R-O-(CH₂-CH₂-O)ₘH (I)
R-COO(CH₂-CH₂-O)ₘH (II)
ausgewählt ist, wobei R eine lineare oder verzweigte, gesättigte oder ungesättigte Kohlenwasserstoffkette mit 10 bis 24 Kohlenstoffatomen ist und m eine ganze Zahl von 8 bis 50 ist.

5. Produkt nach einem der vorhergehenden Ansprüche, wobei die zweite Zusammensetzung B mindestens 0,5 Gew.-% schäumende(s) grenzflächenaktive(s) Mittel bezogen auf das Gesamtgewicht der zweiten Zusammensetzung, beispielsweise 0,5 bis 60 Gew.-%, insbesondere 10 bis 60 Gew.-% umfasst.

6. Produkt nach einem der vorhergehenden Ansprüche, wobei die zweite Zusammensetzung B in der Form eines transparenten wässrigen Gels vorliegt.

7. Produkt nach einem der vorhergehenden Ansprüche, wobei die zweite Zusammensetzung B weiterhin mindestens ein Polymer umfasst, ausgewählt aus oxyalkylenierten nichtionischen Polymeren, kationischen Polymeren und amphoteren Polymeren.

8. Produkt nach einem der vorhergehenden Ansprüche, wobei die erste und/oder die zweite Zusammensetzung eine Viskosität von größer als 2 Pa.s bei Umgebungstemperatur von 20 bis 25 °C und bei Umgebungsdruck aufweist.

9. Produkt nach einem der vorhergehenden Ansprüche, **dadurch gekennzeichnet, dass** es ein Reinigungsprodukt, ein Abschminkprodukt, ein Peelingprodukt, ein Exfoliationsprodukt aufbaut.

10. Einheit von Verpackung und Verteilung der Zusammensetzungen, die ein Produkt nach einem der Ansprüche 1 bis 9 bilden, wobei die Einheit mindestens zwei unabhängige Kompartimente umfasst, die jeweils eine der Zusammensetzungen umfassen, und wobei sie zum Verteilen der beiden Zusammensetzungen getrennt oder im Gemisch ausgelegt ist.

11. Verfahren zum Reinigen und/oder Abschminken für keratinische Substanz(en), umfassend mindestens die folgenden Schritte, die bestehen aus:
a) Bereitstellen einer ersten Zusammensetzung A, die in der Form einer Öl-in-Wasser-Emulsion vorliegt, die durch das Phaseninversions-Temperaturverfahren gemäß der PIT-Technologie erhalten wird, und, in einem physiologisch verträglichen Medium, mindestens 50 Gew.-% von mindestens einem Öl oder einer öligen Phase, bezogen auf das Gesamtgewicht der ersten Zusammensetzung, umfasst, und
b) Bereitstellen einer zweiten Zusammensetzung B, die in der Form eines wässrigen Gels vorliegt, und, in einem physiologisch verträglichen Medium, mindestens ein schäumendes grenzflächenaktives Mittel umfasst,
c) weiterhin extemporanes Inkontaktbringen der ersten Zusammensetzung A mit der zweiten Zusammensetzung B, und
d) Aufbringen des im vorhergehenden Schritt erhaltenen Gemisches auf die keratinische Substanz.

12. Verfahren nach dem vorhergehenden Anspruch, wobei die erste und die zweite Zusammensetzung so sind, wie in den Ansprüchen 1 bis 9 definiert.

13. Verfahren nach einem der Ansprüche 11 oder 12, wobei die Schritte c) und d) gleichzeitig durchgeführt werden.

14. Verfahren nach einem der Ansprüche 11 bis 13, wobei Schritt d) in Gegenwart von Wasser durchgeführt wird.

15. Verfahren nach einem der Ansprüche 11 bis 14, wobei auf Schritt d) ein Abspülschritt mit Wasser der behandelten keratinischen Substanz folgt.
